(19) Europäisches Patentamt
European Patent Office
Office européen des brevets

(11) Publication number: **0 295 842 A1**

(12) **EUROPEAN PATENT APPLICATION**

(21) Application number: 88305363.9

(22) Date of filing: 13.06.88

(51) Int. Cl.⁴: C07D 307/62 , C09D 5/24

(30) Priority: 15.06.87 JP 148376/87
24.06.87 JP 156967/87

(43) Date of publication of application:
21.12.88 Bulletin 88/51

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Matsumura, Koichi
12-9, Terada-cho
Ibaraki Osaka 567(JP)
Inventor: Shimizu, Yoshiaki
4-4, Nikawacho 1-chome
Nishinomiya Hyogo 662(JP)
Inventor: Sugihara, Yoshihiro
4-5, Tamaicho 3-chome
Toyonaka Osaka 560(JP)
Inventor: Mise, Noritoshi
8-4, Teratani-cho
Takatsuki Osaka 569(JP)

(74) Representative: Lewin, John Harvey et al
Elkington and Fife High Holborn House 52/54
High Holborn
London WC1V 6SH(GB)

(54) **Saccharoascorbic acid esters, production and use thereof.**

(57) A saccharoascorbic acid ester or a salt represented by the general formula of

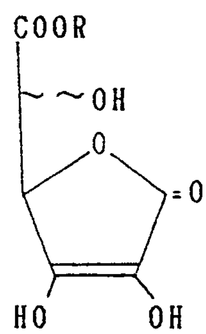

wherein R represents an organic residue of a molecular weight of 15-500, and OH has an S- or an R-configuration, and an electroconductive coating composition which contains such an ester or a salt thereof as an antioxidant together with an electroconductive metal powder.

EP 0 295 842 A1

## Saccharoascorbic Acid Esters, Production and Use Thereof

This invention relates to saccharoascorbic acid esters and salts thereof, and production and use of the same. More particularly, the invention relates to saccharoascorbic acid esters represented by the general formula of

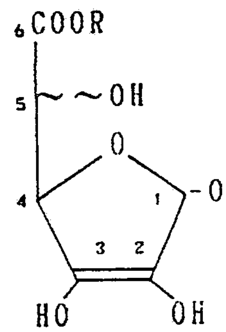

namely, esters of D-glucosaccharoascorbic acid or D-erythrohexo-2-enaro-1,4-lactone, and esters of L-gulosaccharoascorbic acid or L-threo-hexo-2-enaro-1,4-lactone, both being esterified at the 6-position carboxylic group, and salts thereof, and further to a method of producing the same and use of the same.

The invention relates further to an electroconductive coating composition which contains a saccharoascorbic acid ester or a salt thereof.

L-Ascorbic acid, D-erythorbic acid, their derivatives, and L-gulosaccharoascorbic acid (described in U.S. Patent Nos. 2,428,438 and 2,438,251; Carbohydrate Research, 60, 251-258 (1978) ; Vitamine, 56, 117-131 (1982)) are already known to have antioxidant activity.

In the course of intensive investigation of saccharoascorbic acids, their derivatives and use, the present inventors have obtained, as novel compounds, esters of L-gulo- and D-glucosaccharoascorbic acids having the 6-position carboxylic group esterified. The inventors further found that these compounds have excellent antioxidant activity, so that they have various uses, and are in particular useful as an antioxidant in an electroconductive coating composition which contains a metal powder sensitive to air oxidation.

An electroconductive coating composition is used for, for example, surface coating of a synthetic resin casing which contains therein electronic circuit devices. This surface coating renders the surface electroconductive, and prevents the permeation of electromagnetic waves through the casing, and thus erroneous operation of the circuit device.

An electroconductive coating composition is usually composed of an organic solvent, a binder resin such as an acrylic resin, and an electroconductive metal powder such as silver, nickel or copper dispersed therein. It is generally needed that the composition retains not only high workability as a composition but also high electroconductivity as an electroconductive film over a long period of time. It is further needed that the composition is inexpensive.

Thus a variety of electroconductive coating compositions have been recently proposed which contain a less expensive copper powder therein. However, since copper is very sensitive to surface oxidation with the air, an electroconductive film formed therewith is remarkably reduced in electroconductivity with time.

It has been therefore proposed to add a compound which has antioxidant activity to an electroconductive coating composition. There are already known as such an additive, for example, anthranilic acid (Japanese Patent Laid-open No. 58-97892), and a combination of a reducing compound such as hydroquinone and a chelatable compound with copper ions such as acetylacetone. However, the electroconductive film formed with a composition that contains such an additive as above is still found unsatisfactory in a long period stability of electroconductivity.

Meanwhile, an adhesive composition is already known which contains ascorbic acid or an ester thereof as a rust inhibitor (Japanese Patent Publication No. 54-9613). However, since an electroconductive coating composition usually contains an organic solvent as beforedescribed, ascorbic acid which is only slightly soluble therein is not suitable for use as an antioxidant in an electroconductive coating composition. An carboxylic acid ester of ascorbic acid is soluble in many organic solvents, but it is difficult to obtain the ester in a pure form, which makes it difficult to produce a composition having a stable and constant quality.

The present inventors have found that the use of saccharoascorbic acid esters and salts thereof, which have both reducing ability and chelating ability with metal ions, as an antioxidant additive, provides an

electroconductive coating composition which forms a film whose electroconductivity is retained high over a long period of time.

It is, therefore, an object of the invention to provide novel compounds, L-gulo- and D-glucosaccharoascorbic acid esters and salts thereof.

It is another object of the invention to provide a method of producing L-gulo and D-glucosaccharoascorbic acid esters and salts thereof.

It is still an object of the invention to provide an electroconductive coating composition containing the novel saccharoascorbic acid esters or salts thereof.

According to the invention, there is provided a novel saccharoascorbic acid ester, and a salt thereof. The saccharoascorbic acid ester is represented by the general formula of

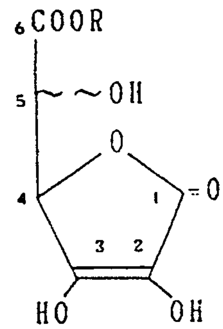

(I)

wherein R represents an organic residue of a molecular weight of 15-500, preferably 15-300, and OH has either an S- or an R-configuration, as designated herein by a wavelike line.

In the general formula (I), R is preferably a hydrocarbon residue of 1-24 carbons, which include an alkyl, an alkenyl or an alkynyl, either linear or branched, a cycloalkyl, an aryl or an arylalkyl. The alkyl of 1-24 carbons includes, for example, methyl, ethyl, n-propyl, isopropyl, n-butyl, isobutyl, sec.-butyl, tert.-butyl, n-pentyl, isoamyl, sec.-amyl, tert.-amyl, neopentyl, n-hexyl, n-heptyl, n-octyl, sec.-octyl, n-nonyl, isononyl, n-decyl, n-undecyl, n-dodecyl, n-tridecyl, n-tetradecyl, n-pentadecyl, n-hexadecyl, n-heptadecyl, n-octadecyl, isooctadecyl, n-nonadecyl, n-eicosyl, n-heneicosyl, n-docosyl, n-tricosyl and n-tetracosyl. These alkyls may have substituents thereon, for example, a halogen such as chlorine, bromine, iodine or fluorine, cyano, hydroxyl, carboxyl or its ester, aminocarbonyl, ether group as represented by -O-alkyl wherein the alkyl is preferably of 1-8 carbons or -O-aryl wherein the aryl is preferably phenyl, acyl as represented by -CO-alkyl wherein the alkyl is preferably of 1-8 carbons, or aroyl represented by -CO-aryl wherein the aryl is preferably phenyl, such as benzoyl.

The alkenyl is preferably of 2-20 carbons, and includes, for example, vinyl, propenyl, butenyl, pentenyl, hexenyl, heptenyl, octenyl, nonenyl, decenyl, undecenyl, dodecenyl, tridecenyl, tetradecenyl, pentadecenyl, heptadecenyl, octadecenyl, nonedecenyl and eicosenyl. These alkenyls may have substituents thereon as beforementioned or an alkyl of preferably 1-3 carbons.

The alkynyl is also preferably of 2-20 carbons, and includes, for example, ethynyl, propynyl, butynyl, pentynyl, hexynyl, heptynyl, octynyl, nonynyl, decynyl, undecynyl, dodecynyl, tridecynyl, tetradecynyl, pentadecynyl, hepadecynyl, octadecynyl, nonadecynyl and eicosynyl. These alkynyls may have substituents thereon as beforementioned or an alkyl of preferably 1-3 carbons.

The cycloalkyl is preferably of 3-8 carbons, and there may be mentioned as examples, cyclopropyl, cyclobutyl, cyclopentyl, cyclohexyl, cycloheptyl and cyclooctyl. The cycloalkyl also may have substituents thereon as beforementioned or an alkyl of preferably 1-3 carbons.

There may be mentioned as the aryl, for example, phenyl, furyl, thienyl, pyridyl or naphthyl, and these aryls may have substituents thereon as beforementioned or an alkyl of preferably 1-3 carbons.

The arylalkyl is exemplified by an alkyl preferably of 1-4 carbons having substituents thereon such as aryl as above mentioned, i.e., phenyl, furyl, thienyl, pyridyl or naphthyl The arylalkyl may have substituents thereon as beforementioned or an alkyl of preferably 1-3 carbons. More specific examples of the arylalkyl include benzyl, phenethyl, 1-phenylethyl, 1-phenylpropyl, 3-phenylpropyl, 1-methyl-3-phenylpropyl and 4-phenylbutyl.

The saccharoascorbic acid ester (I) of the invention may be produced by use of a saccharoascorbic acid as represented by the formula

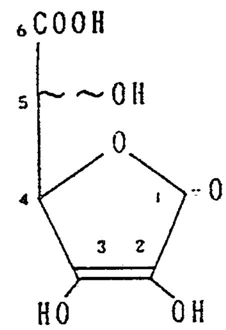

(II)

as a starting material. In the formula (II), the compound having a 5-position OH on the right hand (absolute configuration of R) is L-gulosaccharoascorbic acid. This compound is already known, as described in U.S. Patent No. 2,428,438. However, the compound having a 5-position OH on the left hand (absolute configuration of S) is D-glucosaccharoascorbic acid, and is a novel compound. The D-glucosaccharoascorbic acid may be produced by treating 2-keto-D-glucaric acid or D-arabino-2-hexulosaric acid, or its 2,3-0-acetal or ketal with an acid.

The saccharoascorbic acid esters of the invention may be produced by a variety of methods. Preferred examples of the methods will now be described.

Method (A):

The saccharoascorbic acid ester (I) of the invention is produced by the reaction of the saccharoascorbic acid (II) with an alcohol having the general formula of

R-OH (III)

wherein R is the same as before.

This reaction is one of the most popular ester synthesizing reactions, namely a dehydration reaction between the carboxylic acid and the alcohol. The reaction is an equilibrium reaction, and proceeds in the absence of a catalyst, however, it is preferred that the reaction be carried out in the presence of a catalyst. Any known esterification catalyst may be used, and mineral acids, organic acids or Lewis acids are usually used. More specifically, there may be used, as mineral acids, hydrogen halides such as hydrogen chloride, hydrogen bromide, hydrogen iodide, hydrogen fluoride; perchloric acid; sulfuric acid; fluorosulfuric acid; phosphoric acid or boric acid. There may be used, as organic acids, arenesulfonic acids such as p-toluenesulfonic acid or benzenesulfonic acid; alkanesulfonic acids such as methanesulfonic acid or trifluoromethanesulfonic acid; aliphatic carboxylic acids such as acetic acid or propionic acid; halogenated acetic acids such as trifluoroacetic acid or trichloroacetic acid; or $H^+$-form ion exchange resins. The Lewis acids usable include, for example, boron trifluoride, boron trifluorideether complexes, borontrichloride, boron tribromide, boron triiodide, aluminum chloride, titanium tetrachloride, zinc chloride, stannous chloride and stannic chloride. These acids may be used as they are, or as solutions or suspensions in water or organic solvents, if desired. Further, the acids may be used singly or as a mixture of two or more.

The acids are used usually in amounts of about 0.001-5 %, preferably about 0.01-2 % by weight based on the amount of the saccharoascorbic acid (II) used as a starting material.

The saccharoascorbic acid (II) as a starting material is used usually in the form of a free acid, which may or may not contain water of crystallization. However, the acid may be used in the form of a salt such as an alkali metal salt, e.g., lithium salt, sodium salt or potassium salt; an alkaline earth metal salt, e.g., magnesium salt, calcium salt or barium salt; or an ammonium salt. When the salts are used as a starting material, the salts are reacted with an acid in about stoichiometric amounts or in slight excess amounts, usually up to 1.5 moles per mole of the salts, prior to the esterification reaction, so that the salts are converted to free acids.

The esterification of the saccharoascorbic acid or its salt is carried out usually in a solvent. Any solvent which does not adversely affect the reaction may be used, such as acetone, methyl ethyl ketone, cyclohexanone, hexane, cyclohexane, acetonitrile, propionitrile, benzonitrile, nitromethane, nitroethane, nitrobenzene, dichloromethane, chloroform, carbon tetrachloride, 1,1-dichloroethane, 1,2-dichloroethane, 1,1,1-trichloroethane, 1,1,2-trichloroethane, trichloroethylene, tetrachloroethylene, benzene, toluene, xylene,

formamide, dimethylformamide, dimethylacetamide, dimethyl sulfoxide, hexamethylphsphoramide, sulfolane, dioxane, tetrahydrofuran, ethyl ether, dimethoxyethane, ethylene glycol dimethyl ether, diethylene glycol dimethyl ether, ethyl acetate, or water. These solvents may be used singly or as a mixture. The aforesaid alcohols as a reactant as represented by the general formula (III) may be used also as a solvent.

In the esterification reaction, the alcohol (III) may be used in from equimolar amounts to large excess amounts to the saccharoascorbic acid (II). For instance, when the alcohol is used also as a solvent, the amount may be about 300 times in moles as much as the acid.

Since the esterification reaction is an equilibrium reaction, it is preferred that the water generated in the reaction or the water contained in the reaction mixture be removed from the reaction mixture during the reaction by a conventional method to increase the yield of the esters. The water may be removed by distillation, preferably by azeotropic distillation with a solvent. When azeotropic distillation is employed, the azeotropic vapor may be cooled to separate the water therefrom, and the recovered solvent may be returned to the reaction mixture. Alternatively, the azeotropic vapor may be removed from the reaction mixture, while a dried fresh solvent may be added to the reaction mixture in amounts equal to that of the solvent removed therefrom. However, water only may be removed depending on the alcohol (III) or solvents used when the alcohol (III) or solvents have higher boiling points than water.

The water may also be removed with a dehydrating or drying agent. For example, the azeotropic vapor as it is or after cooling to condensates may be dried over a drying agent such as anhydrous calcium sulfate, anhydrous sodium sulfate, anhydrous magnesium sulfate, molecular sieves, silica gel or alumina, and then returned to a reaction vessel. Orthoformates or 1,1-dialkoxypropane may be used, for example, as a dehydrating agent. A dehydrating agent may be added to the reaction mixture to remove the water therein.

The reaction temperature is usually in the range of from about 0 °C to about 150 °C, preferably from about 20 °C to about 120 °C. The reaction is carried out usually under normal pressures, however, the reaction may be carried out under reduced pressures to accelerate the distillative or azeotropic removal of water from the reaction mixture.

Further, the reaction time may vary depending upon the alcohols (III) used, catalysts and other reaction conditions employed, but it is usually in the range of about 0.5-24 hours, preferably of about 1-15 hours, although not critical.

Method (B):

The saccharoascorbic acid ester (I) of the invention is obtained by the reaction of a saccharoascorbic acid ester having the general formula of

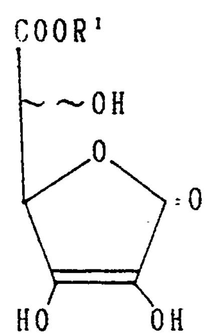

(IV)

wherein R′ represents a hydrocarbon residue of 1-12 carbons, with the aforesaid alcohol (III). This reaction is an ester exchange reaction.

In the general formula (IV), $R^1$ is a hydrocarbon residue of 1-12 carbons, and includes, for example, an alkyl, an alkenyl or an alkynyl, either linear or branched, a cycloalkyl, an aryl or an arylalkyl.

The alkyl of 1-12 carbons includes, for example, methyl, ethyl, n-propyl, isopropyl, n-butyl, isobutyl,

sec.-butyl, tert.-butyl, n-pentyl, isoamyl, sec.-amyl, tert.-amyl, neopentyl, n-hexyl, n-heptyl, n-octyl, sec.-octyl, n-nonyl, isononyl, n-decyl, n-undecyl and n-dodecyl. The alkenyl is of 2-12 carbons, and includes, for example, vinyl, propenyl, butenyl, pentenyl, hexenyl, heptenyl, octenyl, nonenyl, decenyl, undecenyl and dodecenyl. Similarly the alkynyl is of 2-12 carbons, and includes, for example, ethynyl, propynyl, butynyl, pentynyl, hexynyl, heptynyl, octynyl, nonynyl, decynyl, undecynyl and dodecynyl.

The cycloalkyl is preferably of 3-8 carbons, and there may be mentioned as examples, cyclopropyl, cyclobutyl, cyclopentyl, cyclohexyl, cycloheptyl and cyclooctyl.

There may be mentioned as the aryl, for example, phenyl, furyl, thienyl, pyridyl or naphthyl, and the arylalkyl is preferably of 7-10 carbons, and may be exemplified by an alkyl preferably of 1-4 carbons having substituents thereon such as aryl as above mentioned, i.e., phenyl, furyl, thienyl, pyridyl or naphthyl. More specific examples of the arylalkyl include benzyl, phenethyl, 1-phenylethyl, 1-phenylpropyl, 3-phenylpropyl, 1-methyl-3-phenylpropyl and 4-phenylbutyl.

The ester compound (IV) as a starting material may be produced by the reaction of the saccharoascorbic acid (II) with an alcohol represented by the general formula of

$R^1$-OH

wherein $R^1$ is the same as before, in accordance with the method (A) as described hereinbefore.

Similarly to the esterification reaction (A) as herein before mentioned, this reaction is also an equilibrium reaction, and is carried out usually in the presence of a catalyst, usually mineral acids, organic acids or Lewis acids. More specifically, there may be used, as mineral acids, hydrogen halides such as hydrogen chloride, hydrogen bromide, hydrogen iodide or hydrogen fluoride; perchloric acid; sulfuric acid; fluorosulfuric acid; phosphoric acid or boric acid. There may be used, as organic acids, arenesulfonic acids such as p-toluenesulfonic acid or benezenesulfonic acid; alkanesulfonic acids such as methanesulfonic acid or trifluoromethanesulfonic acid; aliphatic carboxylic acids such as acetic acid or propionic acid; halogenated acetic acids such as trifluoroacetic acid or trichloroacetic acid; or $H^+$-form ion exchange resins. The Lewis acids usable include, for example, boron trifluoride, boron trifluoride-ether complexes, boron trichloride, boron tribromide, boron triiodide, aluminum chloride, titanium tetrachloride, zinc chloride, stannous chloride and stannic chloride. These acids may be used as they are, or as solutions or suspensions in water or organic solvents, if desired. Further, the acids may be used singly or as a mixture of two or more.

The acids are used usually in amounts of about 0.001-5 %, preferably about 0.01-2 % by weight based on the amount of the saccharoascorbic acid ester (IV) used as a starting material.

The reaction is carried out either in a solvent or without a solvent. Any solvent which does not adversely affect the reaction may be used, such as hexane, cyclohexane, acetonitrile, propionitrile, benzonitrile, nitromethane, nitroethane, nitrobenzene, dichloromethane, chloroform, carbon tetrachloride, 1,1-dichloroethane, 1,2-dichloroethane, 1,1,1-trichloroethane, 1,1,2-trichloroethane, trichloroethylene, tetrachloroethylene, benzene, toluene, xylene, formamide, dimethylformamide, dimethylacetamide, dimethyl sulfonxide, hexamethylphsphoramide, sulfolane, dioxane, tetrahydrofuran, ethyl ether, dimethoxyethane, ethylene glycol dimethyl ether, diethylene glycol dimethyl ether, ethyl acetate, or water. These solvents may be used singly or as a mixture. The aforesaid alcohols as represented by the general formula (III) may be used as a solvent, too.

In the reaction of the method (B), ester exchange reactions take place between the saccharoascorbic acid ester (IV) and the alcohol (III) in the presence of an acid catalyst, to produce equilibrium mixtures. Therefore, it is preferred that the alcohol (III) be used in large excess amounts, as a reactant but also as a solvent, usually in amounts of about 300 times in moles as much as the saccharoascorbic acid ester used, to increase the yield of the desired saccharoascorbic acid ester. As a further method, it is also preferred that a by-produced alcohol be removed from the reaction mixture when the by-produced alcohol have low boiling points, thereby to shift the equilibrium to the side of desired saccharoascorbic acid ester. In this method, the alcohol (III) may be used in amounts ranging from an equivalent to 5 times as much as equivalent to the saccharoascorbic acid ester (IV).

The reaction temperature is usually in the range of from about 0 °C to about 150 °C, preferably from about 20 °C to about 120 °C. The reaction may be carried out under reduced pressures to accelerate the distillative removal of the by-produced alcohol, i.e., $R^1$-OH, from the reaction mixture.

The reaction time may vary depending upon the saccharoascorbic acid ester (IV) and the alcohols (III) used, catalysts used and other reaction conditions employed, but it is usually in the range of about 0.5-24 hours, perferably of about 1-15 hours, although not critical.

Method (C):

The saccharoascorbic acid ester (I) of the invention is obtainable by reacting a saccharoascorbic acid salt having the general formula of

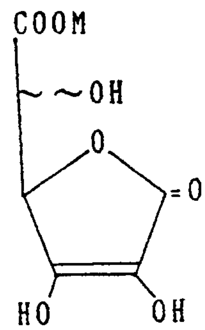

(V)

wherein M represents an alkali metal, an alkaline earth metal, an ammonium, a substituted ammonium or pyridinum, either with a compound having the general formula of

R-X (VI)

wherein R is the same as before, and X represents a halogen, an alkylsulfonyloxy or an arylsulfonyloxy, or with a compound having the general formula of

$(RO)_2SO_2$ (VII)

wherein R is the same as before.

In the general formula (V), M is an alkali metal such as lithium, sodium or potassium; an alkaline earth metal such as magnesium, calcium or barium; an ammoinium or a substituted ammonium ($>N^+<$) preferably having at least one substituent of an alkyl of 1-6 carbons, a cycloalkyl, preferably cyclohexyl, or an aryl, preferably benzyl or phenyl; or having at least one 5- or 6-membered carbon or heterocyclic ring formed with the nitrogen atom of the ammonium, optionally further with nitrogen or oxygen atoms contained therein; or pyridinium. More specifically, there may be mentioned as examples of the substituted ammonium, methylammonium, ethylammnoium, propylammnoium, butylammonium, pentylammonium, hexylammonium, anili nium, benzylammonium, dimethylammonium, diethylammonium, dipropylammnoium, dibutylammonium, dipentylammonium, dihexyl- ammonium, dianilinium, piperidinium, morpholinium, pyridazinium, pyrrolidinium, dibenzylammonium, trimethylammonium, triethylammnoium, tripropylammnoium, tributylammonium, tripentylammonium, trihexylammonium, tribenzylammonium, tetramethylammonium, tetraethylammnoium, tetrapropylammnoium, tetrabutylammonium, tetrapentylammonium, tetrahexylammonium, trimethylphenylammonium, trimethylbenzylammonium, triethylphenylammonium, triethylbenzylammonium, tripropylphenylammonium, tripropylbenzylammonium, tributylphenylammonium or tributylbenzylammonium.

In turn, in the general formula (VI), X is a halogen such as chlorine, bromine, iodine, or fluorine; an alkylsulfonyloxy such as methanesulfonyloxy, ethanesulfonyloxy; trichloromethanesulfonyloxy or trifluoromethanesulfonyloxy; or an arylsulfonyloxy such as benzenesulfonyloxy, p-toluenesulfonyloxy, o-toluenesulfonyloxy, p-chlorobenzenesulfonyloxy, o-nitrotoluenesulfonyloxy, m-nitrotoluenesulfonyloxy or p-nitrotoluenesulfonyloxy.

The saccharoascorbic acid salt (V) used as a starting material in the method may be produced by a known method as, for example, described in Vitamine, 56, 117-131 (1982), or any method similar thereto. The salt (V) may also be produced by the reaction of the saccharoascorbic acid (II) with an appropriate base material exemplified by, for example, hydroxides, carbonates or hydrogen carbonates of alkali metals or alkaline earth metals, or pyridine, tertiary amines or substituted ammonium hydroxides having a partial structure of $\equiv N^+OH^-$. More specifically with hydroxides, carbonates or hydrogen carbonates of alkali metals or alkaline earth metals, there may be mentioned as examples, lithium hydroxide, lithium hydrogen carbonate, lithium carbonate, sodium hydroxide, sodium hydrogen carbonate, sodium carbonate, potassium hydroxide, potassium hydrogen carbonate, potassium carbonate, magnesium hydroxide, magnesium carbonate, calcium hydroxide, calcium carbonate, barium hydroxide and barium carbonate. These base materials may be used in amounts of from about equivalent to 1.5 times as much as an equivalent to the saccharoascorbic acid (II).

The esterification reaction according to the method (C) is carried out usually in the presence of a solvent. Any solvent which does not adversely affect the reaction may be used, but a polar solvent is preferred, such as acetonitrile, propionitrile, benzonitrile, formamide, dimethylformamide, dimethylacetamide, dimethyl sulfoxide, sulfolane, hexamethylphosphoramide, acetone, methyl ethyl ketone, dioxane, tetrahydrofuran, ethylene glycol dimethyl ether, diethylene glycol dimethyl ether, or water. These solvents may be used singly or as a mixture.

The compounds (VI) or (VII) may be used in amounts of about 1-20 moles, preferably in amounts of about 1.2-10 moles, per mole of the saccharoascorbic acid salt (V) used.

The reaction temperature is usually in the range of from about 0 °C to about 100 °C, preferably from about 10 °C to about 80 °C. The reaction time may vary depending upon the saccharoascorbic acid salt (V) and the other reactants used, but it is usually in the range of about 0.5-24 hours, preferably of about 1-15 hours, although not critical.

Method (D):

The saccharoascorbic acid ester (I) of the invention is produced by subjecting a saccharoascorbic acid ester having the general formula of

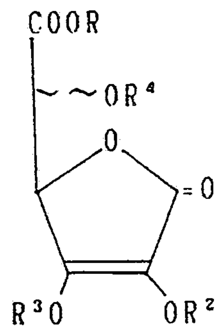

(VIII)

wherein R is the same as before; $R^2$ represents a hydrogen or a protective group of a hydroxyl group; $R^3$ represents a protective group of a hydroxyl group; and $R^4$ represents a hydrogen, an acyl or a protective group of a hydroxyl group, to an elimination reaction of the protective group and the acyl group.

In the general formula of (VIII), the hydroxyl protective group represented by $R^2$, $R^3$ and $R^4$ are those which are eliminated by reduction. Such protective groups are already known, and include, for example, benzyl and a substituted benzyl, preferably with a lower alkoxy, a nitro, a halogen or a cyano, such as p-methoxybenzyl, o-nitrobenzyl, p-nitrobenzyl, p-chlorobenzyl, p-bromobenzyl or p-cyanobenzyl. A diphenylmethyl is also an example of a preferred protective group. The acyl group is preferably of 1-12 carbons, and is most preferably a lower acyl of 1-7 carbons, which includes, for example, formyl, acetyl, propionyl, butyryl, valeryl, isovaleryl, pyvaloyl or benzoyl.

In this method, the acyl group may be first eliminated, and then the hydroxyl protective group may be eliminated; or the hydroxyl protective group may be first eliminated, and then the acyl group may be eliminated. When the $R^2$, $R^3$ and $R^4$ are all hydroxyl protective groups, they are all eliminated in a single elimination reaction.

The acyl group of 5-position carbon may be eliminated by hydrolysis thereof with an acid or a base. When the acyl group of 5-position carbon only is to be hydrolyzed in high yields while the ester group of the 6-position carbon is to be remained unaffected, it is preferred that the reaction be carried out under conditions as mild as possible. The 5-position acyl group may be selectively eliminated since the 5-position acyl is usually more readily hydrolyzed, although somewhat depending upon the ester structure of the 6-position carbon and the acyl structure $R^4$ of the 5-position carbon.

The acid used in the hydrolysis is not specifically limited, but any acid usable in hydrolysis in general may be used also in this method. Therefore, among others are usable mineral acids and organic acids. More specifically, there may be used, as mineral acids, hydrogen halides such as hydrogen chloride, hydrogen bromide, hydrogen iodide or hydrogen fluoride; perchloric acid; sulfuric acid; fluorosulfuric acid, phosphoric acid or boric acid. There may be used as organic acids, arenesulfonic acids such as p-toluenesulfonic acid or benzenesulfonic acid; alkanesulfonic acids such as methanesulfonic acid or trifluoromethanesulfonic acid; aliphatic carboxylic acids such as acetic acid or propionic acid; halogenated acetic acids such as trifluoroacetic acid or trichloroacetic acid; or $H^+$-form ion exchange resins. These acids may be used as they are, or as solutions or suspensions in water or organic solvents, if desired. Further, the acids may be used singly or as a mixture of two or more.

The base used in the hydrolysis also is not specifically limited, but any base usable in hydrolysis in general may be used in this method. Therefore, there may be mentioned as examples of the bases usable, hydroxides, carbonates or hydrogen carbonates of alkali metals or alkaline earth metals, such as lithium hydroxide, lithium hydrogen carbonate, lithium carbonate, sodium hydroxide, sodium hydrogen carbonate, sodium carbonate, potassium hydroxide, potassium hydrogen carbonate, potassium carbonate, magnesium hydroxide, magnesium carbonate, calcium hydroxide, calcium carbonate, barium hydroxide or barium carbonate; organic bases such as pyridine, or primary, secondary or tertiary amines.

Any solvent which does not adversely affect the reaction may be used in the reaction, but a solvent which has a high affinity with water is preferred, such as acetone, methyl ethyl ketone, methanol, ethanol, n-propanol, isopropanol, tert.-butanol, acetonitrile, propionitrile, dioxane, tetrahydrofuran, ethyl ether, ethylene glycol dimethyl ether, diethylene glycol dimethyl ether, ethyl acetate, dimethylformamide, dimethylacetamide, dimethyl sulfoxide or hexamethylphosphoramide. Water also may be used as a solvent. These solvents may be used singly or as a mixture.

The reaction temperature is usually in the range of from about -20°C to about 120°C, preferably from about 0°C to about 100°C, although depending upon the compound (VIII) used and other reaction conditions employed. Similarly the reaction time may vary depending upon the compond (VIII) used and other reaction conditions employed, but it is usually in the range of about 1-10 hours.

As above described, the compound (VIII) which has an acyl group $R^4$ at 5-position carbon can be converted to a compound represented by the general formula of

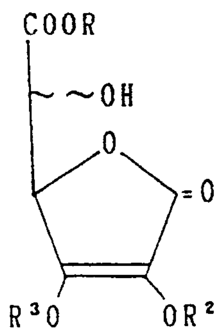

(IX)

wherein R, $R^2$ and $R^3$ are the same as before, by deacylation of the 5-position acyl group by hydrolysis.

Then, the elimiation of the hydroxyl protective group at the 2- and 3-position carbon provides the saccharoascorbic acid ester (I) of the invention. The elimiation is usually accomplished by catalytic reduction using hydrogen in the presence of a catalyst such as palladium chloride, platinum oxide or platinum black. These may be supported on activated carbon, as exemplified by Pd/C, alumina, or silica gel.

The catalytic reduction reaction is carried out usually in a solvent, such as methanol, ethanol, n-propanol, isopropanol, acetic acid, acetonitrile, propionitrile, dioxane, tetrahydrofuran, ethyl ether, 1,2-dimethoxyethane, ethylene glycol dimethyl ether, chloroform, dichloromethane, benzene, toluene, or water. These solvents may be used singly or as a mixture.

The reaction is carried out usually at temperatures in the range of about 10-100°C, either under normal or increased pressures.

On the other hand, when the hydroxyl protective groups $R^2$ and $R^3$ at the 2- and 3-position carbons, respectively, are first to be eliminated, prior to the deacylation, the method which is used to convert the compound (IX) to the saccharoascorbic acid ester (I) may be employed as it is. Namely, the catalytic reduction of the ester (IX) with hydrogen in a reaction solvent in the presence of a catalyst provides a compound having an acyl group $R^4$ at the 5-position carbon and having the general formula of

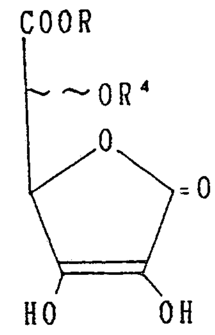

(X)

wherein R is the same as before.

Then, the application to the above compound (X) of the hydrolysis method which is used to convert the compound (VIII) to the compound (IX) can eliminate the acyl group $R^4$ selectively, thereby to provide the saccharoascorbic acid ester (I).

When $R^2$, $R^3$ and $R^4$ in the compound (VIII) are all hydroxyl protective groups, the aforesaid catalytic reduction of such compounds provides the ester (I) in a single step.

As above set forth, the saccharoascorbic acid ester (I) of the invention is obtainable by the method (A), (B), (C) or (D), among others, usually in the form of free acids, while the method (D) may provide the ester (I) in the form of salts as well.

The saccharoascorbic acid ester (I) of the invention may be isolated and purified by a conventional method. Namely, after the reaction, solvents and low boiling temperature materials are removed in a conventional manner from the reaction mixture, and the resultant residue is subjected to extraction, chromatography using, for example, silica gel, polystyrene resins or activated carbon, distillation or recrystallization. The saccharoascorbic acid ester (I) may be isolated either an anhydrous or hydrated compound.

The saccharoascorbic acid ester (I) in the form of free acids may be converted to the corresponding salts by the reaction of the acid with a base material such as an alkali metal oxide, an alkali metal hydroxide, an alkali metal carbonate, an alkali metal hydrogen carbonate, an alkaline earth metal oxide, an alkaline earth metal hydroxide or an alkaline earth metal carbonate, an amine or an ammonium hydroxide. The ester (I) in the form of free acids may be converted to salts also by putting the ester in the form of free acids into contact with a cation exchange resin substituted with an alkali metal ion, an alkaline earth metal ion or an ammonium ion.

If desired, after the reaction, there is added to the reaction mixture, without isolating the saccharoascorbic acid ester, an appropriate base material such as an alkali metal oxide, an alkali metal hydroxide, an alkali metal carbonate, an alkali metal hydrogen carbonate, an alkaline earth metal oxide, an alkaline earth metal hydroxide or an alkaline earth metal carbonate, an amine or an ammonium hydroxide, or the reaction mixture is put into contact with a cation exchange resin having an appropriate alkali metal ion, an alkaline earth metal ion or an ammonium ion substituted, thereby to convert the ester(I) directly to salt forms. The salts are then isolated and purified in a conventional manner such as recrystallization, reprecipitation or the like.

The saccharoascorbic acid ester (I) and a salt thereof may contain water of crystallization.

The salt of the saccharoascorbic acid ester (I) of the invention includes, for example, an alkali metal salt such as lithium, sodium or potassium salt; an alkaline earth metal salt such as magnesium, calcium or barium salt; and an ammonium, a substituted ammonium or a pyridinium salt.

There may be mentioned as examples of substituted ammonium salts, methylammonium, ethylammonium, propylammnoium, butylammonium, pentylammonium, hexylammonium, anilinium, benzylammonium, dimethylammonium, diethylammonium, dipropylammnoium, dibutylammonium, dipentylammonium, dihexylammonium, dianilinium, piperidinium, morpholinium, pyridazinium, pyrrolidinium, dibenzylammonium, trimethylammonium, triethylammnoium, tripropylammnoium, tributylammonium, tripentylammonium, trihexylammonium, tribenzylammonium, tetramethylammonium, tetraethylammnoium, tetrapropylammnoium, tetrabutylammonium, tetrapentylammonium, tetrahexylammonium, trimethylphenylammonium, trimethylbenzylammonium, triethylphenylammonium, triethylbenzylammonium, tripropylphenylammonium, tripropylbenzylammonium, tributylphenylammonium or tributylbenzylammonium salt.

The aforementioned saccharoascorbic acid ester (VIII) is also a novel compound, and may be produced

in accordance with the scheme shown below:

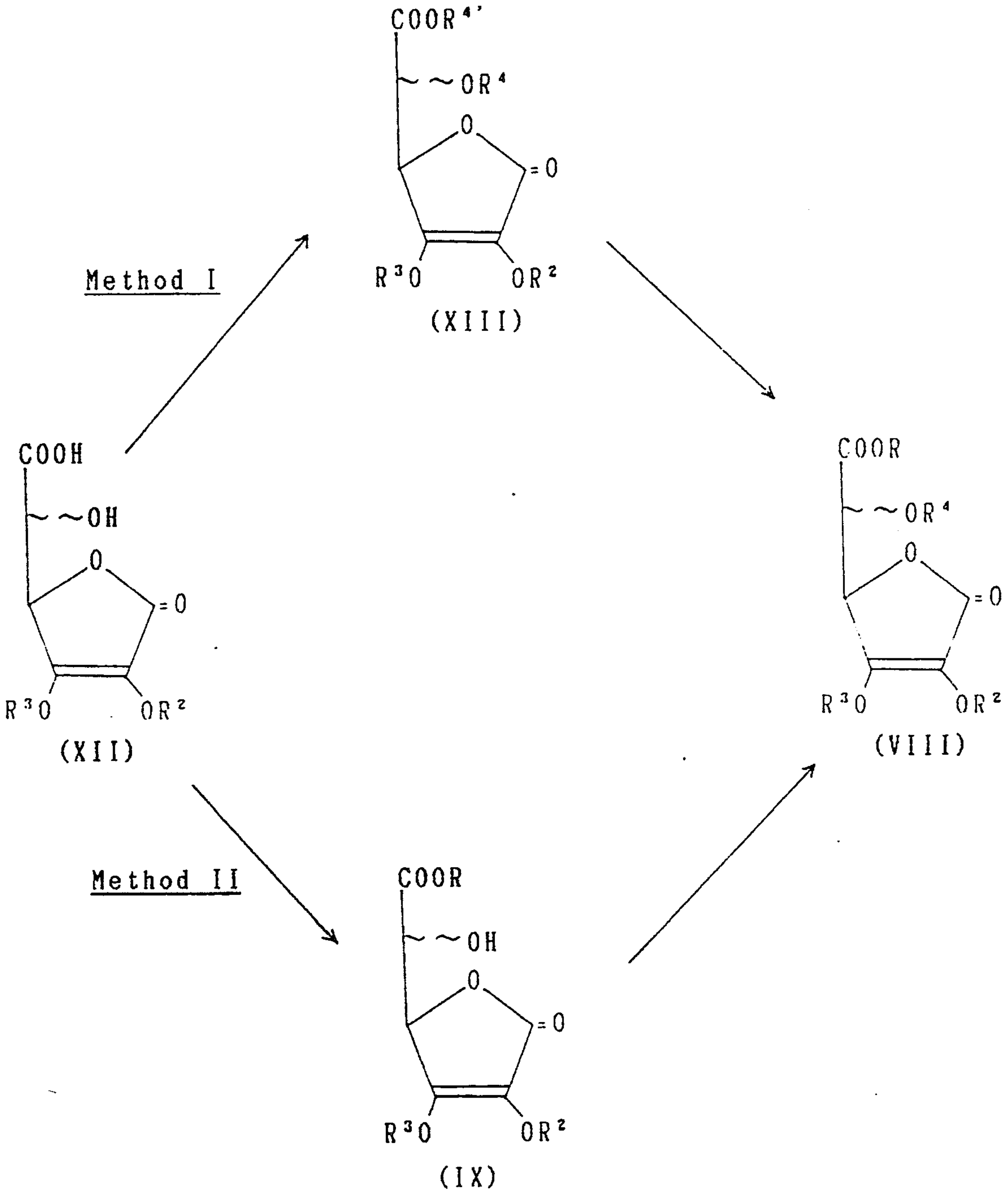


(XIII) (XII) (VIII) (IX)

The ester (VIII) may be produced either by Method I or II.

<u>Method I</u>

The ester (XIII) is a compound wherein $R^2$, $R^3$ and $R^4$ are the same as before, and $R^{4'}$ represents a hydrogen or is the same as $R^4$ which is the same as beforementioned. The ester (XIII) wherein $R^4$ is an acyl may be produced by application of a conventional acylation reaction to the compound (XII). Namely, the reaction of the compound (XII) with a conventional acylation agent such as an aliphatic or aromatic acid anhydride, inclusive of a mixed acid anhydride, of 1-12 carbons, or an acid halide thereof such as an acid chloride, acid bromide, acid iodide or acid fluoride, in the presence of an acid or a base catalyst, provides

the acylated compound (XIII).

In the acylation reaction, however, two kinds of compounds are produced depending upon the reaction conditions or reagents used since the starting material (XII) is an α-hydroxy-carboxylic acid. One is a compound in which only the 5-position hydroxyl has been acylated, i.e., the compound (XIII) in which $R^{4'}$ is hydrogen, and the other is a compound in which both of the 5-position hydroxyl and the 6-position carboxyl have been acylated to form a mixed acid anhydride at the 6-position carbon, i.e., the compound (XIII) in which both of $R^{4'}$ and $R^4$ are acyl groups. Although depending upon the reaction conditions, methods of after-treatment, or the stability of the compound (XIII), either or both of the compound (XIII) in which $R^{4'}$ is hydrogen or acyl may be isolated, if needed. Further if needed, the $COOR^{4'}$ (wherein $R^{4'}$ is acyl) at the 6-position carbon only is hydrolyzed to carboxyl, to provide the compound (XIII) wherein $R^{4'}$ is hydrogen.

On the other hand, the compound (XIII) in which $R^4$ is a hydroxyl protective group which is eliminated by a reduction reaction may be produced by a known benzylation reaction of the compound (XII). For instance, the reaction of the compound (XII) with an appropriate benzyl halide such as benzyl chloride, benzyl bromide or benzyl iodide, in the presence of a dehydrohalogenation agent in the absence or presence of a solvent, provides the compound (XIII) in which $R^4$ and $R^{4'}$ are hydroxyl protective groups which are capable of being eliminated by a reduction reaction.

In this reaction, a base material is used as a dehydrohalogenation agent, such as sodium hydride, sodium hydroxide, sodium hydrogen carbonate, sodium carbonate, potassium hydride, potassium carbonate, potassium-hydrogen carbonate, pyridine, or tertiary amines, e.g., triethylamine, tripropylamine, diethylpropylamine or 4-dimethylaminopyridine. Silver oxide or silver carbonate are also usable as a dehydrohalogenation agent. When the reaction is carried out in a solvent, there may be usually used as a solvent, acetone, methyl ethyl ketone, acetonitrile, propionitrile, nitromethane, nitroethane, nitrobenzene, ethyl acetate, dichloromethane, chloroform, carbon tetrachloride, dioxane, tetrahydrofuran, 1,2-dimethoxyethane, ethylene glycol dimethyl ether, diethylene glycol dimethyl ether, diethyl carbonate, formamide, dimethylformamide, dimethyl sulfoxide, sulfolane, or hexamethylphosphoramide.

The reaction temperature is usually in the range of from about 0 °C to about 120 °C, althogh depending upon the reactants and reagents used and the reaction conditions employed. Similarly the reaction time may vary, however, it is usually in the range of about 1-24 hours.

The application of conventional esterification reactions to the thus obtained compound (XIII) provides the compound (VIII).

More specifically, the compound (XIII) in which $R^{4'}$ is hydrogen is esterified by a conventional method to provide the compound (VIII). Such conventional esterification methods include, for example, a so-called direct esterification method in which the compound (XIII) is reacted with the alcohol (III) in the presence of an acid catalyst; a method via carboxylates in which the compound (XIII) is reacted with the aforesaid compounds, RX (VI) or $(RO)_2SO_2$ (VII) in the presence of a base material; a method in which the compound (XIII) is reacted with the aforesaid alcohol (III) in the presence of a dehydration condensation agent exemplified by dicyclohexylcarbodiimide; a method in which the compound (XIII) is reacted with an olefin compound exemplified by isobutylene in the presence of an acid catalyst; a method in which the compound (XIII) is reacted with an O-alkylating agent exemplified by diazomethane or orthoformates; or the compound (XIII) is first converted to active ester compounds, which is then subjected to alcoholysis by the alcohol (III).

When the compound (XIII) has an acyl group as $R^{4'}$, it corresponds to an active ester compound as above mentioned. Therefore, the reaction of the compound (XIII) with the alcohol (III) in the presence of a base catalyst provides the saccharoascorbic acid ester (VIII). When the compound (XIII) has a hydroxyl protective group as $R^{4'}$, the application of ester exchange reaction thereto provides the compound (VIII).

Method II:

In the steps of from the compound (XII) wherein $R^2$ and $R^3$ are the same as before to the compound (IX) wherein, R, $R^2$ and $R^3$ are the same as before, a conventional esterification method may be adoptable. Such conventional esterification methods include, for instance, a direct esterification method in which the compound (XII) is reacted with the alcohol (III) in the presence of an acid catalyst; a method via carboxylates in which the compound (XII) is reacted with the aforesaid compounds, RX (VI) or $(RO)_2SO_2$ (VII) in the presence of a base material; a method in which the compound (XII) is reacted with the aforesaid alcohol (III) in the presence of a dehydration-condensation agent exemplified by dicyclohexylcarbodiimide; a method in which the compound (XII) is reacted with an olefin compound exemplified by isobutylene in the presence of an acid catalyst; or a method in which the compound (XII) is reacted with an O-alkylating agent exemplified by diazomethane or orthoformates.

In the step of from the compound (IX) to the compound (VIII) wherein $R^4$ is not hydrogen, conventional acylation methods are applicable to the step when $R^4$ is an acyl group. The acylation may be carried out by the reaction of the compound (IX) with an acylating agent such as an acid anhydride of an aliphatic carboxylic acid of 1-12 carbons or an aromatic carboxylic acid, inclusive of a mixed acid anhydride, or an acid halide such as acid chloride, acid bromide, acid iodide or acid fluoride in the presence of an acid catalyst or a basic condensation agent.

On the other hand, when the compound (XII) has a hydroxyl protective group as $R^4$ which is eliminated by a reduction reaction, the reaction of thereof with a benzyl halide such as benzyl chloride, benzyl bromide or benzyl iodide, in the presence of a base material or a dehydrohalogenation agent such as silver oxide or silver carbonate, provides the compound (VIII).

The aforesaid compound (XII) used as a starting material in the production of the compound (VIII) as above described is also a novel compound. The compound (XII) may be produced in accordance with the steps shown below:

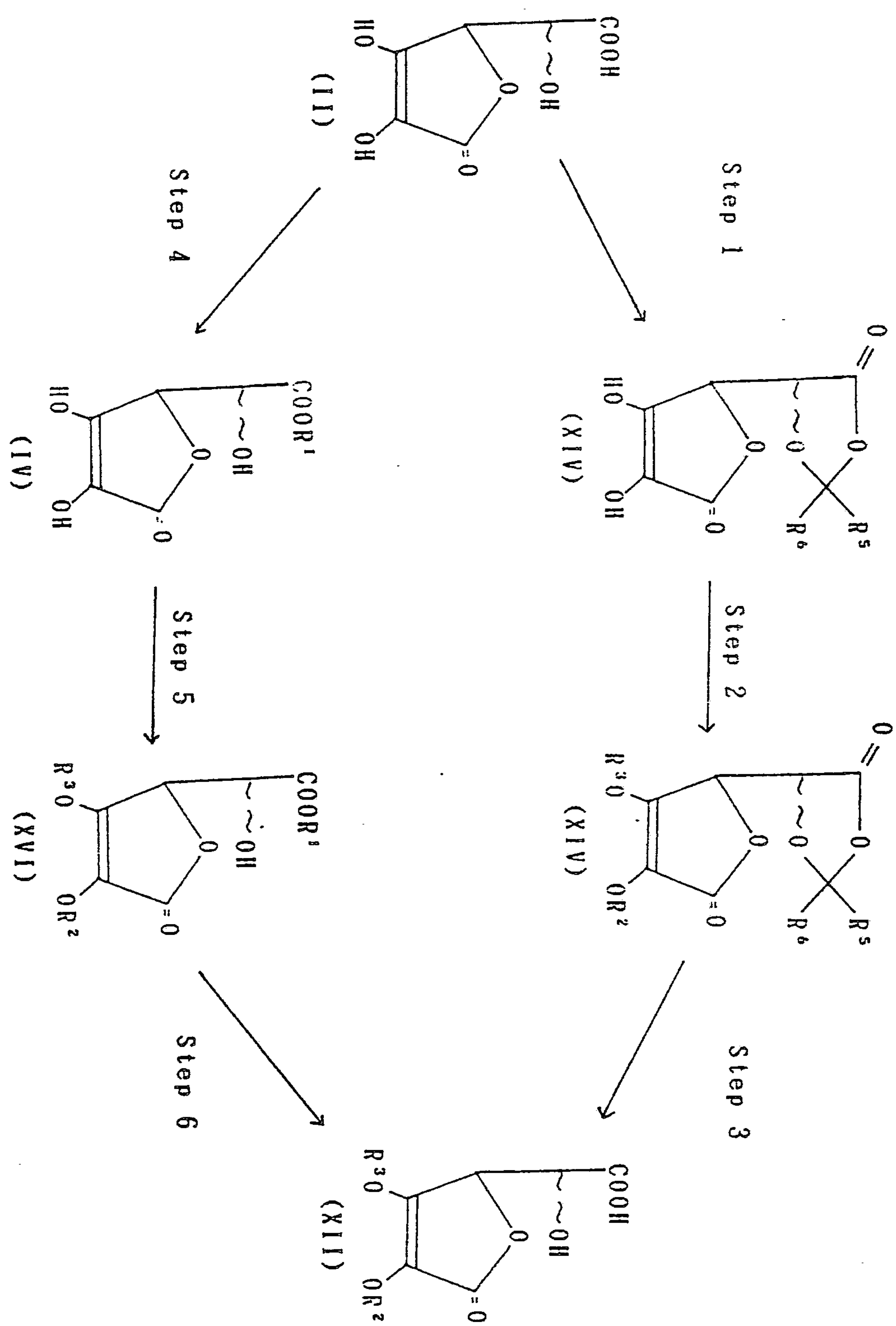


(II) (XIV) (XIV) (IV) (XVI) (XII)

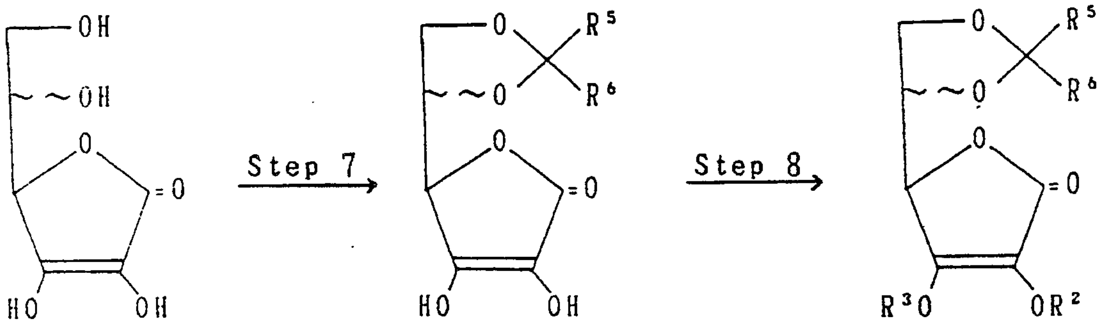


(XVII) (XVIII) (XIX)

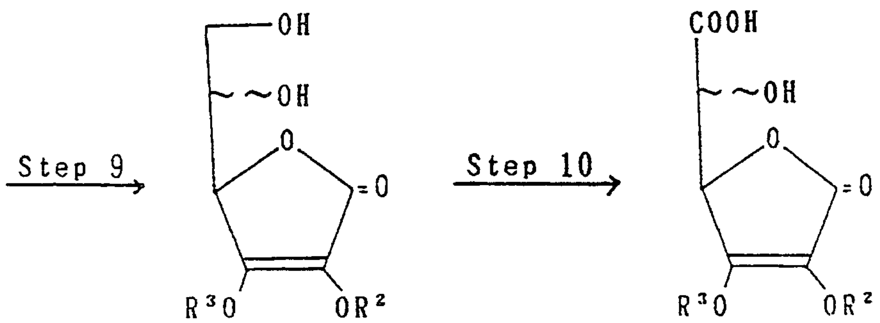


(XX) (XII)

The step 1 is a ketal or an acetal formation reaction of the saccharoascorbic acid (II) for the production of the compound (XIV) in which $R^5$ and $R^6$ are the same or different from each other, and represent independently hydrogen, methyl, ethyl or phenyl, or $R^5$ and $R^6$ combinedly form a divalent polymethylene group represented by $-(CH_2)_n$ wherein n is 4 or 5. Namely, the saccharoascorbic acid (II) is reacted with an aldehyde or a ketone, or a ketal or an acetal of an aldehyde or a ketone, such as formaldehyde, acetaldehyde, acetone, propionaldehyde, methyl ethyl ketone, diethyl ketone, cyclopentanone, cyclohexanone or benzaldehyde in the presence of an acid catalyst, to provide the compound (XIV).

The reaction is carried out usually in the presence of a solvent. Any solvent which does not adversely affect the reaction may be used, such as acetonitrile, propionitrile, benzonitrile, nitromethane, nitroethane, nitrobenzene, chloroform, carbon tetrachloride, 1,1-dichloroethane, 1,2-dichloroethane, hexane, cyclohexane, benzene, toluene, xylene, dioxane, tetrahydrofuran, 1,2-dimethoxyethane, ethylene glycol dimethyl ether, diethyl carbonate, dimethylformamide, or dimethyl sulfoxide. If desired, the aforementioned ketones or aldehydes, or their ketals or acetals, may be used also as a solvent. These solvents may be used singly or as a mixture.

The acid catalyst usable includes, for example, mineral acids, such as hydrogen halides, e.g., hydrogen chloride, hydrogen bromide, hydrogen iodide or hydrogen fluoride; perchloric acid; sulfuric acid; fluorosulfuric acid; phosphoric acid or boric acid; organic acids such as arenesulfonic acids, e.g., p-toluenesulfonic acid or benzenesulfonic acid; alkane-sulfonicacids, e.g., methanesulfonic acid or trifluoromethane-sulfonic acid; halogenated acetic acids, e.g., trifluoroacetic acid or trichloroacetic acid; or $H^+$- form ion exchange resins; or Lewis acids such as boron trifluoride, boron trichloride, boron tribromide, boron triiodide, aluminum chloride, titanium tetrachloride, zinc chloride, stannous chloride or stannic chloride.

The reaction temperature is usually in the range of from about 0-100°C, and the reaction time is usually in the range of about 1-24 hours.

The step 2 is a reaction for the production of the compound (XV) wherein $R^2$, $R^3$, $R^5$ and $R^6$ are the same as before from the compound (XIV). In this step the compound (XIV) is reacted with a benzyl halide such as benzyl chloride, benzyl bromide or benzyl iodide, in the presence of a base material or a

dehydrohalogenation agent such as silver oxide or silver carbonate. The benzyl halide is usually used in amounts of about 1-8 moles, preferably in amounts of about 1-6 moles, per mole of the compound (XIV). The dehydrohalogenation agent is used usually in amounts of about 1.5-20 moles per mole of the compound (XIV).

The reaction is carried out usually in a solvent. Any solvent which does not adversely affect the reaction may be used. Such solvents include, for example, acetone, methyl ethyl ketone, acetonitrile, propionitrile, nitromethane, nitroethane, nitrobenzene, ethyl acetatre, dioxane, tetrahydrofuran, 1,2-dimethoxyethane, ethylene glycol dimethyl ether, diethylene glycol dimethyl ether, diethyl carbonate formamide, dimethylformamide, dimethyl sulfoxide, sulfolane, or hexamethylphosphoramide. These solvents may be used singly or as a mixture of two or more.

The reaction temperature is usually in the range of from about 0-120° C. The reaction time is usually in the range of about 1-24 hours, although depending upon the reactants, dehydrohalogenation agent and solvents used, and the reaction conditions employed.

The step 4 from the compound (II) to the compound (IV) is described bereinbefore with regard to the production of the latter. The step 5 from the compound (IV) to the compound (XVI) may be carried out in the same manner as in the production of the compound (XVI) from the compound (XIV), as described hereinbefore.

The step 3 from the compound (XV) to the compound (XII) may be carried out by hydrolysis of the acetal or ketal group at the 5- and 6-position carbons under acidic or alkaline conditions. Similarly to the above, the step 6 from the compound (XVI) to the compound (XII) may be carried out by hydrolysis of the ester group at the 6-position carbon under acidic or alkaline conditions.

In the above hydrolysis reactions, there may be used as an alkaline material, for example, sodium hydroxide, sodium hydrogen carbonate, sodium carbonate, potassium hydroxide, potassium carbonate, potassium hydrogen carbonate, barium hydroxide, ammonia, sodium methoxide, or sodium ethoxide. There may be used as an acid, for example, hydrogen halides such as hydrogen chloride, hydrogen bromide, hydrogen iodide or hydrogen fluoride, perchloric acid, sulfuric acid, phosphoric acid, boric acid, acetic acid, trifluoroacetic acid, trichloroacetic acid, p-toluenesulfonic acid, methanesulfonic acid or $H^+$-form ion exchange resins. The alkaline or acid materials may be used as they are, or as a solution or a suspension in a solvent. Further they may be used singly or as mixture of two or more. The amount of the alkaline or acid materials used may range from a catalytic amount to large excess where they are used as a solvent as well. Accordingly, the amount may preferably range from about 0.01 -500 % by weight based on the compound (XV) and (XVI), respectively.

The hydrolysis reaction is carried out usually in a solvent, which includes, for instance, water, acetonitrile, methanol, ethanol, n-propanol, isopropanol, n-butanol, tert.-butanol, acetone, methyl ethyl ketone, benzene, toluene, xylene, dioxane, tetrahydrofuran, ethyl ether, 1,2-dimethoxyethane, ethylene glycol dimethyl ether, diethylene glycol dimethyl ether, formamide, dimethylformamide, or dimethyl sulfoxide. These solvents may be used singly or as a mixture of two or more.

The reaction temperature is usually in the range of from about -20° C to 100° C, while the reaction time is usually in the range of about 1-10 hours, although depending upon the reactants, reagents and solvents used, and the reaction conditions employed.

Meanwhile, the step 7 is for the production of the compound (XVIII) wherein $R^5$ and $R^6$ are the same as before, and an acetal or ketal formation reaction of the hydroxyls at the 5- and 6-position carbons either of L-ascorbic acid (XVII) in which the 5-position hydroxyl is on the right hand or has an absolute configuration S, or erythorbic acid (XVII) in which the 5-position hydroxyl is on the left hand or has an absolute configuration R. This acetal or ketal formation reaction may be accomplished by a method known per se, as described, for example, in Japanese Patent Laid-open No. 60-69079.

The step 8 from the compound (XVIII) to the compound (XIX) may be carried out in the same manner as in the step 5 as hereinbefore described. The step 9 from the compound (XIX) to the compound (XX) may be carried out by hydrolysis in the same manner as in the step 3 or 6 as hereinbefore described.

For the step 10, namely, for the selective oxidation of the thus obtained compound (XX) to the compound (XII), Heyns' oxidation method may be preferably employed. This oxidation method is a catalytic oxidation with the air or oxygen in the presence of a catalyst such as platinum or palladium which may be supported on activated carbon, alumina or silica gel. The reaction is carried out in the presence of a solvent such as water, dioxane, tetrahydrofuran, 1,2-dimethoxyethane, ethylene glycol dimethyl ether, diethylene glycol dimethyl ether, acetone, methyl ethyl ketone, cyclohexanone, acetonitrile, propionitrile or dimethylformamide. These solvents may be used singly or as a mixture of two or more.

In this oxidation reaction, the reaction mixture becomes acidic as the reaction proceeds since the product (XII) is an acid. Therefore, it is preferred that an alkaline material, such as sodium hydroxide,

sodium hydrogen carbonate, sodium carbonate, potassium hydroxide, potassium hydrogen carbonate or potassium carbonate, either as it is or as a solution, be added dropwise to the reaction mixture in accordance with the progress of the reaction to maintain the mixture nearly neutral, for example, at a pH of about 5.5-8.5.

The reaction temperature is usually in the range of from about 10 °C to 100 °C, while the reaction time is usually in the range of about 2-48 hours, although depending upon the reactants and solvents used, and the reaction conditions employed.

According to the above oxidation reaction, the compound (XII) is obtained usually as a salt of an alkaline material used in the neutralization. If needed, the salt may be converted into free acids (XII) by the application of mineral acids such as hydrochloric acid or sulfuric acid or $H^+$-form cation exchange resins to the salt.

The saccharoascorbic acid ester (I) and a salt thereof of the invention have an excellent antioxidant activity on account of the reductive endiol group in the molecule, and therefore they are useful as antioxidants in a variety of fields. For instance, the ester (I) and a salt thereof of the invention improve the durability of an electroconductive coating composition when being contained therein as an antioxidant. They are also useful as antioxidants for food, and oil and fat. Further the ester (I) and a salt thereof of the invention serve as a deodoring agent or deoxygen agent in combination with metal salts such as iron salts, and as a corrosion inhibitor in sealants such as butyl sealants.

Further, the saccharoascorbic acid ester (I) and a salt thereof of the invention are useful as intermediates in organic synthesis. In particular, the ester (I) has two optically active carbons at the 4- and 5-position, so that it is useful in the synthesis of a variety of optically active products such as natural compounds, medicines, agricultural chemicals or functional compounds all of increasing importance.

In addition, the saccharoascorbic acid ester derivative (VIII) but also the compounds (IX), (XII), (XIII), (XIV), (XV) and (XVI) as set forth as intermediates for the production of the compound (VIII) are all novel compounds, and they are useful as intermediates for the production of the saccharoascorbic acid ester (I) of the invention.

The electroconductive coating composition of the invention will now be described.

The electroconductive coating composition of the invention comprises: an organic solvent, a resin, an electroconductive metal powder, and saccharoascorbic acid ester or a salt thereof.

In the invention, the saccharoascorbic acid esters are especially preferred as an antioxidant since they have in general a high solubility in many organic solvents but also they are chemically stable. Further, the esters are readily obtained in a pure form contrary to ascorbic acid esters. Ascorbic acid has two endiol type hydroxyls and two non-endiol type hydroxyls, so that it is very difficult to esterify the non-endiol type hydroxyls selectively with a carboxylic acid, resulting in the formation of mixtures of esters. However, since the saccharoascorbic acid has only one carboxylic group therein, the esterification thereof with an alcohol readily provides a single ester in a high yield, not a mixture of esters.

The antioxidant activity of the saccharoascorbic acid esters derives from reducing ability and chelating ability with metal ions, as hereinbefore described, so that any ester may be used as an antioxidant in the electroconductive coating composition of the invention. However, methyl, ethyl, isopropyl, isobutyl, tert.-butyl, octyl, decyl, cetyl, oleyl, stearly, phenyl, benzyl, allyl or propargyl esters may be preferred.

The electroconductive coating composition of the invention contains the saccharoascorbic acid ester, an ester or a mixture of these, in amounts of about 0.01-10 % by weight, preferably of about 0.05-5 % by weight, most preferably of about 0.1-3 % by weight, based on the weight of an electroconductive metal powder in the composition.

A variety of electroconductive metals are usable in the invention, and there may be mentioned as examples, powders of copper, silver, aluminum, nickel, cromium, or alloys of two or more of these metals. Although the metal powders usable in the invention are not limited to the above exemplified, however, a copper powder may be used as one of the most preferred metal powders since the saccharoascorbic acid esters prevent very effectively the surface oxidation of copper powders. Electroconductive substances such as carbon black may be also usable in place of an electroconductive metal powder. The electroconductive substances, either metal powders or carbon black, are contained usually in amounts of about from 10 % to about 80 % by weight based on the composition.

The electroconductive coating composition of the invention contains a resin which may be any one used in the conventional electroconductive coating compositions, and includes among others, for example, acrylic resins, polyester resins, epoxy resins, urethane resins, oil base alkyd resins, or emulsions of synthetic rubbers or resins. The resin is contained in the composition usually in amounts of about 20-90 % by weight based on the composition.

The electroconductive coating composition of the invention further contains an organic solvent. The

solvent usable also is not specifically limited, however, ketones, esters, chlorinated hydrocarbons, hydrocarbons, alcohols, ethers, or mixtures of two or more are usually used.

The invention will now be described with reference to examples, which follows.

Reference Example 1

A PYG medium containing 0.5 % of peptone, 0.5 % of yeast extract, 1.0 % of glucose and 0.1 % of $K_2HPO_4$ was placed in a 200 ml capacity Erlenmeyer flask, and steam-sterilized at 120 °C for 20 minutes. This flask was inoculated with one loopful of fresh cells of Pseudomonas aeruginosa IFO 3448 gtown at 28 °C for 2 days on a slant medium prepared by supple menting PYG medium with 2.0 % of agar. Cultivation was conducted at 30 °C for 24 hours with rotation for shaking to give a seed culture.

After preliminary pH adjustment to 7.0 with NaOH and bacterial filtration using a 0.45 micron filter, monopotassium D-glucarate (by Sigma) was added to PYG medium to a concentration of 1 %. To a 200 ml capacity Erlenmeyer flask containing 20 ml of this medium was transferred 1 ml of the above seed culture, and shake culture was performed at 30 °C for 24 hours. According to high performance liquid chromatography using a sulfonated polystyrene gel column (by Shimadzu, SCR-101H column of 7.9 mm x 30 cm; mobile phase: diluted sulfuric acid, ph 2.2; flow rate: 0.5 ml/min.; detector: differential diffractometer), the thus obtained culture broth was found to contain 9.02 mg/ml of 2-keto-D-glucaric acid.

The cells were removed from 590 ml of the culture broth by centrifugation to give 580 ml of a supernatant. Cations in the supernatant were removed by passing through a column of Amberlite cation exchange resin IR120 ($H^+$-form, 200 ml) and washing with 150 ml of deionized water, and then the supernatant was decolorized by passing through a column of activated carbon (70 ml) and washing with 50 ml of deionized water.

The thus decolorized effluent in amounts of 780 ml was adjusted at pH to 6.5 with $Ca(OH)_2$, filtered to remove turbidity and the concentrated to about 20 ml under reduced pressures, so that white amorphous crystals formed in the concentrate. The crystals were collected on a glass filter, washed with small amounts of cold water, methanol and then ethyl ether, and dried under reduced pressures to give 5.04 g of dicalcium 2-keto-D-glucarate 3.5 hydrate. The analytical data for the crystals were shown below.

Melting point: 152-157 °C (decomposed)

Elemental analysis (%) for $C_6H_6O_8Ca \bullet 3.5H_2O$

Calculated:

C, 23.30; H, 4.24; Ca, 12.96

Found:

C, 23.15; H, 4.18; Ca, 14.00

Specific rotation:

$[\alpha]_D^{25}$ = -11.0° (c = 1.075 %, 0.1N HCl, immediately after dissolution)

$[\alpha]_D^{25}$ = +9.0° (c = 1.075 %, 0.1N HCl, after equilibration)

IR spectrum (maximum absorptions, $cm^-$, KBr):

3590, 3500, 3400-2700 (br), 1650, 1600, 1430, 1380, 1360, 1300, 1250, 1240, 1220, 1125, 1095, 1065, 1040, 1005, 995, 955, 900, 840, 800, 765, 725.

It was found that the dicalcium 2-keto-D-glucarate varied in the amount of hydrated water. The elemental analysis study showed that the salt ranged from monohydrate to tetrahydrate.

Reference Example 2

To a stirred suspension of 5.0 g (0.0167 mole) of dicalcium 2-keto-D-glucarate trihydrate in 250 ml of acetone was added gradually 2.5 ml of concentrated sulfuric acid. After completion of the addition, stirring was continued for another 3 hours. Insoluble materials were then filtered off using about 20 g of Hyflo Super Cel (Johns-Manville, U.S.A.) as filter aids, and then washed with about 1000 ml of acetone.

The filtrate and the washings were combined and concentrated under reduced pressures, and the crystalline precipitates were collected by filtration, washed with small amounts of ethyl acetate and then dried in a desiccator, to provide 3.80 g of 2,3-0-isopropylidene-2-keto-D-glucaric acid as colorless needles. The yield was found 91.7 %. The analytical data were shown below.

Melting point: 180-210 °C (recrystallized from acetone/ether, decomposed)

Elemental analysis (%) for $C_9H_{12}O_8$

Calculated:

C, 43.56; H, 4.87
Found:
C, 43.57; H, 4.99
IR spectrum (maximum absorptions, $cm^{-1}$, KBr):
3400, 3300-2800, 1750, 1690.
$^1H$-NMR ($d_6$-DMSO, $\delta$ ):
1.30(s, 3H), 1.39(s, 3H), 4.35-4.7(m, 3H), 8-10(br, 3H).
$^{13}C$-NMR ($D_2O$, $\delta$ ):
25.3(q), 25.5(q), 76.7(d), 86.7(d), 111.8(s), 116.7(s), 170.4(s), 173.0(s).

Reference Example 3

A mixture of 24.8 g (0.1 mole) of 2,3-0-isopropylidene-2-keto-D-glucaric acid and 50 ml of concentrated hydrochloric acid was stirred at 50°C for 20 minutes. The resultant reaction mixture was concentrated to dryness under reduced pressures, 10 ml of distilled water were added to the residue, and the resultant solution was passed through a column having 50 ml of a special grade activate carbon for chromatography (Shirasagi, registered trademark, by Takeda Chemical Industries, Ltd.) packed with the aid of distilled water.

After being eluted with water, the elute was concentrated under reduced pressures. To the residue was added a small amount of dichloromethane, and the resultant insoluble materials were collected by filtration and dried, to provide 19.0 g of crude D-glucosaccharoascorbic acid (D-erythro-hex-2-enaro-1,4-lactone) hydrate. The purity and the yield were found 98.5 % and 90.9 %, respectively. Recrystallization from dried acetonitrile provided pure D-glucosaccharoascorbic acid. The analytical data were shown below.
Melting point: 188-189°C (decomposed)
Elemental analysis (%) for $C_6H_6O_7$
Calculated:
C, 37.91; H, 3.16
Found:
C, 37.80; H, 3.21
IR spectrum (maximum absorptions, $cm^{-1}$, KBr):
3580, 3500, 3400-3000, 1770, 1720, 1690, 1590.
$^1H$-NMR ($d_6$-DMSO, $\delta$ ):
4.42(d, 1H), 4.95(d, 1H), 6.5-9.5(br, 2H), 9.5-13(br, 2H).
$^{13}C$-NMR ($d_6$-DMSO, $\delta$ ):
69.5(d, 5-position C), 77.2 (d, 4-position C), 118.9 (s, 2-position C), 152.3(s, 3-position C), 170.5(s, 1-position C), 171.7(s, 6-position C).

Reference Example 4

A suspension of 500 g of crude dicalcium 2-keto-D-glucarate, which was found to contain 85.1 % or 1.417 mole of dicalcium salt as trihydrate ($C_6H_6O_8Ca{\bullet}3H_2O$) as determined by high performance liquid chromatography, in 1500 ml of distilled water, was stirred at room temperatures while there were added thereto dropwise slowly 214.9 g (2.126 mole) of 97 % sulfuric acid, and after the completion of the addition, the mixture was stirred overnight at room temperatures.

Th resultant insoluble materials were filtered off and washed with about 1000 ml of distilled water. The washings and the filtrate were combined, and concentrated under reduced pressures on a water bath at 55°C to about an amount of 300 ml. After cooling, the resultant insoluble materials were filtered off. The filtrate was warmed for 8 hours on a water bath at 55°C, and concentrated under reduced pressures. To the concentrate were added 200 ml of distilled water and the insoluble material formed was filtered off.

The thus obtained solution was passed through a column having 300 ml of a special grade activated carbon for chromatography (Shirasagi, registered trademark by Takeda Chemical Industries, Ltd.) packed with the aid of distilled water. The elution was carried out with distilled water. The eluate was concentrated under reduced pressures, and the precipitated crystals were collected by filtration, and dried to give the first crude crystals.

The filtrate was again passed through an activated carbon column, and the eluate was concentrated under reduced pressures, and the precipitated crystals were collected by filtration, and dried to give the second crude crystals. The filtrate was further treated in the same manner as above, to provide the third

crude crystals.

The first, second and third crude crystals were combined and mixed together, to provide 247 g of crude D-glucosaccharoascorbic acid monohydrate. The purity and the yield were found 96.8 % and 81.1 %, respectively.

Pure D-glucosaccharoascorbic acid monohydrate was obtained by recrystallization from distilled water. The analytical data were shown below.

Melting point: 134-138 °C

Elemental analysis (%) for $C_8H_6O_8$:

Calculated:

C, 34.63; H, 3.87

Found:

C, 34.52; H, 3.89

IR spectrum (maximum absorptions, $cm^{-1}$, KBr):

3580, 3500, 3400-3000, 1770, 1720, 1690, 1590.

In order to remove water of crystallization from D-glucosaccharoascorbic acid monohydrate, recrystallizing from anhydrous organic solvents or drying under reduced pressures may be employed. However, azeotropic dehydration is preferred, as described below.

An amount of 100 ml of acetonitrile was added to 10.0 g of D-glucosaccharoascorbic acid monohydrate. The acetonitrile was distilled under normal pressures from the mixture while acetonitrile in amounts equal to the acetonitrile distilled was added to the mixture continuously, and in this manner 250 ml of acetonitrile were distilled in total. With the distillation of acetonitrile, anhydrous D-glucosaccharoascorbic acid began to precipitate. After the distillation of acetonitrile, the precipitates were collected by filtration and dried to provide 8.8 g of D-glucosaccharoascorbic acid in a yield of 96.3 %. This product was completely identical with the melting point, IR spectrum, $^1$H-NMR spectrum, $^{13}$C-NMR spectrum, and retention time in high performance liquid chromatography.

Reference Example 5

D-isoascorbic acid was reacted with acetone in accordance with the description in Japanese Patent Laid-open No. 60-69079 to provide 5,6-O-isopropylidene-D-isoascorbic acid.

Melting point: 167-169 °C (recrystallyzed from acetonitrile, decomposed)

Elemental analysis (%) for $C_9H_{12}O_6$

Calculated:

C, 50.00; H, 5.60

Found:

C, 50.10; H, 5.85

IR spectrum (maximum absorptions, $cm^{-1}$, KBr):

3550-3300, 1760, 1665, 1650.

$^1$H-NMR ($d_6$-DMSO, $\delta$ ):

1.32(s, 3H), 4.20-4.55(m, 1H), 4.82(d, J=3Hz, 1H), ca. 9(br, 2H).

Reference Example 6

An amount of 86.5 g of 5,6-O-isopropylidene-D-isoascorbic acid was dissolved in 400 ml of dimethyl sulfoxide, and to the resultant solution were added gradually 110.6 g of potassium carbonate under stirring. An amount of 106.3 g of benzyl chloride was added dropwise to the mixture and stirred at room temperatures for 24 hours.

After the completion of the reaction, 2 liters of water were added to the reaction mixture, and the mixture was extracted with dichloromethane three times with 1 liter in total. The extract was washed with water and dried over sodium sulfate. The solvent was distilled off from the extract, and the residue was subjected to silica gel chromatography using chloroform as a solvent, to provide 88.3 g of 2,3-di-O-benzyl-5,6-O-isopropylidene-D-isoascorbic acid as an oily material in a yield of 55.7 %.

Elemental analysis (%) for $C_{23}H_{24}O_6$

Calculated:

C, 69.68; H, 6.10

Found:
C, 69.50; H, 6.18
$^1$H-NMR ($CDCl_3$, δ ):
1.31(s, 3H), 1.40(s, 3H), 3.6-4.9(m, 2H), 4.1-4.4(m, 1H), 4.70(d, 1H), 5.0-5.3 (m, 4H), 7.1-7.4(m, 10H).

Reference Example 7

An amount of 1 liter of 0.1N hydrochloric acid was added to 39.6 g of 2,3-di-O-benzyl-5,6-O-isopropylidene-D-isoascorbic acid, and the mixture was heated at 80 °C for 2 hours. The reaction mixture was extracted with chloroform twice each with 0.5 liter. The extract was washed with water and dried over sodium sulfate. The solvent was distilled off, and the residue was subjected to silica gel chromatography using chloroform as a solvent to provide 29.4 g of 2,3-di-O-benzyl-D-isoascorbic acid as an oily material in a yield of 82.5 %.
IR spectrum (maximum absorptions, $cm^{-1}$, liquid film)
3600-3100, 1760, 1670.
$^1$H-NMR ($CDCl_3$, δ ):
3.4-4.2(m, 5H), 4.7(d, 1H), 5.0(s, 2H), 5.15(s, 2H), 7.1-7.4(m, 10H).

Reference Example 8

To a mixture of 15.0 g of D-glucosaccharoascorbic acid, 20.55 g of 2,2-dimethoxypropane and 150 ml of acetone were added three drops of concentrated sulfuric acid, and the mixture was stirred at room temperatures for 4 hours. After the completion of the reaction, a small amount of pyridine (about ten drops) was added to the mixture, and low boiling temperature materials were distilled off. The residue was then subjected to silica gel chromatography using ethyl acetate as a solvent, and the solvent was removed from the eluate to provide solid materials. The solid materials were recrystallized from acetone/dichloromethane (1/10) to provide 16.1 g of 5,6-O-isopropylidene-D-glucosaccharoascorbic acid in a yield of 88.7 %.
Melting point: 162-163 °C
Elemental analysis (%) for $C_9H_{10}O_7$
Calculated:
C, 46.96; H, 4.38
Found:
C, 46.84; H, 4.32
IR spectrum (maximum absorptions, $cm^{-1}$, KBr)
3300 3200, 1775, 1750, 1700, 1670.
$^1$H-NMR ($d_6$-DMSO, δ ):
1.58(s, 6H), 4.97(s, 2H).
The OH group was too broad to determine.

Reference Example 9

To a mixture of 0.40 g of L-gulosaccharoascorbic acid, 1.09 g of 2,2-dimethoxypropane and 10 ml of acetone were added one drop of concentrated sulfuric acid, and the resultant mixture was stirred at room temperatures for 3 hours. After the completion of the reaction, four drops of pyridine were added to the mixture, and low boiling temperature materials were distilled off. The residue was then subjectd to silica gel chromatography using ethyl acetate as a solvent, and the solvent was removed from the eluate. The resultant product was recrystallized from ethyl acetate/dichloromethane (1/10) to provide 0.231 g of 5,6-O-isopropylidene-L-gulosaccharoascorbic acid crystals in a yield of 47.7 %.
Melting point: 158-159 °C
Elemental analysis (%) for $C_9H_{10}O_7$
Calculated:
C, 46.96; H, 4.38
Found:
C, 46.42; H, 4.33
IR spectrum (maximum absorptions, $cm^{-1}$, KBr)

3500-3100, 1765, 1705
$^1$H-NMR ($d_6$-DMSO, δ ):
1.47(s, 3H), 1.55(s, 3H), 4.95(m, 2H).
The OH group was too broad to determine.

Reference Example 10

A mixture of 3.0 g of D-glucosaccharoascorbic acid, 60 ml of cyclohexanone dimethylacetal and three drops of concentrated sulfuric acid was stirred at room temperatures overnight. Low boiling temperature materials were distilled off from the reaction mixture, and the residue was subjected to silica gel chromatography using dichlomethane/ethyl acetate as a solvent, and the solvent was removed from the eluate by distition. The resultant product was recrystallized from dichloromethane/n-hexane to provide 1.53 g of 5,6-O-cyclohexylidene-D-glucosaccharoascorbic acid 0.5 hydrate in a yield of 34.7 %.
Melting point: 80-85 °C
Elemental analysis (%) for $C_{12}H_{14}O_7 \bullet 0.5H_2O$:
Calculated:
C, 51.61; H, 5.41
Found:
C, 51.48; H, 5.18
IR spectrum (maximum absorptions, $cm^{-1}$, KBr)
3500-3100, 1770, 1690.
$^1$H-NMR ($d_6$-DMSO, δ ):
1.20-2.00(m, 10H), 5.06(s, 2H), ca. 8.5 (br. 1H), ca. 11.1(br. 1H).

Reference Example 11

An amount of 2.00 g of 4,5-O-isopropylidene-D-glucosaccharoascorbic acid was dissolved in 15 ml of dimethyl sulfoxide. The the resultant solution were added 1.20 g of potassium carbonate, and then 1.49 g of benzyl bromide dropwise, and the mixture was stirred at room temperatures for 1 hour.

After the completion of the reaction and removal of the remaining insoluble salts by filtration, 200 ml of water were added to the filtrate, which was then extracted with dichloromethane three times. The extract was washed with water four times, dried and concentrated under reduced pressure. The residue was subjected to silica gel chromatography using ethyl acetate/n-hexane (1/1) as a solvent to provide 1.67 g of 3-O-benzyl-5,6-O-isopropylidene-D-glucosaccharoascorbic acid as a pasty material in a yield of 59.9 %.

The product was found to crystallize in part in ether/n-hexane (1/4) to give 0.55 g of crystals.
Melting point: 137-139 °C
IR spectrum (maximum absorptions, $cm^{-1}$, KBr)
3430, 1805, 1770, 1705.
$^1$H-NMR ($CDCl_3$, δ ):
1.55(s, 6H), 4.81(d, 1H, J=2Hz), 5.00(d, 1H, J=2Hz), 5.40(br, OH), 5.51(s, 2H), 7.38(s, 5H).
Mass spectrum (m/e)
320 ($M^+$)

Reference Example 12

An amount of 10.00 g of 5,6-O-isopropylidene-D-glucosaccharoascorbic acid was dissolved in 130 ml of dimethyl sulfoxide. To the resultant solution were added 13.2 g of potassium carbonate, and then 11.0 g of benzyl chloride dropwise, and the mixture was stirred at room temperatures for 16 hours.

After completion of the reaction, the remaining insoluble salts were removed by filtration, and 800 ml of water were added to the filtrate. The filtrate was then extracted with dichloromethane three times. The extract was washed with water four times, dried and concentrated under reduced pressures. The residue was subjected to silica gel chromatography using ethyl acetate/n-hexane (1/2) as a solvent, to provide 6.22 g of 2,3-di-O-benzyl-5,6-O-isopropylidene-D-glucosaccharoascorbic acid as an oily material in a yield of 34.9 %.
IR spectrum (maximum absorptions, $cm^{-1}$, liquid film)

1800-1760, 1670.
$^1$H-NMR ($CDCl_3$, δ ):
1.53(s, 6H), 4.77(d, 1H, J=2Hz), 4.96(d, 1H, J=2Hz), 5.14(s, 2H), 5.19(s, 1H), 5.23(s, 1H), 7.07-7.43(m, 10H).
Mass spectrum (m/e)
410 ($M^+$), 395.

## Reference Example 13

An amount of 3.0 g of 5,6-O-isopropylidene-D-glucosaccharoascorbic acid was dissolved in 20 ml of dimethyl sulfoxide. To the resultant solution were added 4.49 g of potassium carbonate, and the mixture was stirred at room temperatures to liberate carbon dioxide. After about 5 minutes, 4.48 of benzyl bromide was added dropwise to the reaction mixture, and then the mixture was stirred at room temperatures for 2 hours.

After completion of the reaction, the insoluble materials formed were removed by filtration, and the filtrate was poured into about 30 ml of ice water, followed by extraction with about 200 ml of dichloromethane. The extract was washed with water, dried over anhydrous sodium sulfate, and concentrated by removing the solvent by distillation under reduced pressures. The residue was then subjected to silica gel chromatography using ethyl acetate/n-hexane (1/2) as a solvent, to provide 2.36 g of 2,3-di-O-benzyl-5,6-O-isopropylidene-D-glucosaccharoascorbic acid as an oily material in a yield of 44.5 %.

## Reference Example 14

An amount of 10.0 g of 2,3-di-O-benzyl-D-isoascorbic acid was dissolved in 300 ml of dioxane/water (1/2), and there were added thereto 10 g of 5 % Pd/C, followed by heating at 60 °C. The air was bubbled into the mixture at a rate of 900 ml/min. while an aqueous solution of $NaHCO_3$ was added to the mixture through a pH controller to maintain the mixtureat a pH of about 7.

After the reaction for 4 hours, the catalyst was removed from the reaction mixture by filtration, and washed with a small amount of dioxane/water, and the washings were combined with the filtrate. The filtrate was concentrated under reduced pressures, and the resultant precipitates were collected by filtration, and washed with a small amount of ethyl acetate, to provide 5.4 g of white powders.

The powders were dissolved in 200 ml of water, and the solution was washed with 60 ml of ethyl acetate. Diluted hydrochloric acid was added to an aquous layer to adjust the pH to about 2-3, and the aqueous layer was extracted with ethyl acetate twice each with 120 ml. The extract was washed with water, dried over anhydrous sodium sulfate, and concentrated by removing the solvent by distillation under reduced pressures. The resultant solids were recrystallized from hot water to provide 3.66 g of 2,3-di-O-benzyl-D-glucosaccharoascorbic acid in a yield of 35.2 %.
Melting point: 123-124 °C (decomposed)
IR spectrum (maximum absorptions, $cm^{-1}$, KBr):
3400, 1770, 1740, 1680.
$^1$H-NMR ($CDCl_3$, δ ):
4.67(d, 1H), 5.06(s, 3H), 5.15(s, 2H), ca. 6.5(br, 2H), 7.1-7.4(m, 10H).
$^{13}$C-NMR ($d_6$-DMSO, δ ):
69.1(d), 72,9(t), 73.9(t), 76.6(d), 121.4(s), 127.3(d), 128.2, 128.4(d), 128.5, 128.7(d), 135.6(s), 136.2(s), 156.8(s), 168.8(s), 171.0(s).

## Reference Example 15

An amount of 22.2 g of methyl D-glucosaccharoascorbate monohydrate was dissolved in 200 ml of dimethyl sulfoxide, and to the resultant solution were added gradually 18.0 g of potassium carbonate under stirring. An amount of 27.9 g of benzyl chloride was added dropwise to the mixture and stirred at room temperature for 24 hours.

After completion of the reaction, 500 ml of ice water were added to the reaction mixture, and the mixture was extracted with 2 liters of ether, and further with 500 ml of ethyl acetate. The extracts were combined together, washed with water, dried over anhydrous sodium sulfate, and concentrated by removing

the solvent by distillatrion under reduced pressures. The residue was subjected to silica gel chromatography using chloroform as a solvent to provide 24.1 g of methyl 2,3-di-O-benzyl-D-glucosaccharoascorbate as an oily material in a yield of 62.6 %.

IR spectrum (maximum absorptions, $cm^{-1}$, liquid film):

3450, 1760, 1690, 1675.

$^1$H-NMR ($CDCl_3$, $\delta$ ):

3.63(s, 3H), 4.6-4.8(m, 1H), 5.05-5.3(m, 5H), ca. 6.5 (br, 1H), 7.2-7.6(m, 10H).

Reference Example 16

An amount of 56.15 g of methyl 2,3-di-O-benzyl-D-glucosaccharoascorbate was added to a mixture of 110 ml of 2N hydrochloric acid and 200 ml of acetonitrile, and then refluxed for 8 hours.

After completion of the reaction, the reaction mixture was concentrated to dryness. A small amount of n-hexane/ethyl acetate was added to the residue, the remaining insoluble materials were collected by filtration, and dried, to give 38.2 g of 2,3-di-O-benzyl-D-glucosaccharoascorbic acid in a yield of 70.6 %.

Reference Example 17

An amount of 4.07 g of 2,3-di-O-benzyl-5,6-O-isopropylidene-D-glucosaccharoascorbic acid was heated at 60° C in a mixture of 20 ml of water/acetic acid (1/1) for 1.5 hours.

After completion of the reaction, the reaction mixture was concentrated under reduced pressures, and the residue was recrystallized from ethyl acetate/n-hexane to provide 3.50 g of 2,3-di-O-benzyl-D-glucosaccharoascorbic acid in a yield of 94.0 %.

Reference Example 18

An amount of 7.13 g of 2,3-di-O-benzyl-L-ascorbic acid was dissolved in 250 ml of a mixture of dioxane and water (1/2), and there were added thereto 7 g of 5 % Pd/C, followed by heating at 60° C. The air was bubbled into the mixture at a rate of 900 ml/min. while an aqueous solution of $NaHCO_3$ was added to the mixture through a pH controller to maintain the mixture at a pH of about 7.

After the reaction for 5 hours, the catalyst was removed from the reaction mixture by filtration, and washed with a small amount of dioxane/water, and the washings were combined with the filtrate. The filtrate was concentrated under reduced pressures, and the resultant precipitates were collected by filtration, and washed with a small amount of ethyl acetate, to provide 4.8 g of colorless powders.

The powders were dissolved in 150 ml of water, and the solution was washed with 60 ml of ethyl acetate. The resultant aqueous layer was separated, and there was added thereto diluted hydrochloric acid to adjust the pH of the aqueous layer to about 1-2. Then the aqueous layer was extracted with ethyl acetate twice each with 120 ml. The extract was washed with water, dried over anhydrous sodium sulfate, and concentrated by removing the solvent of distillation under reduced pressures. The resultant residue was purified by silica gel chromatography using chloroform as a solvent to provide 4.20 g of 2,3-di-O-benzyl-L-gulosaccharoascorbic acid as an oily material in a yield of 56.7 %.

Elemental analysis (%) for $C_{20}H_{18}O_7 \bullet 0.2H_2O$

Calculated:

C, 64.24; H, 4.96

Found:

C, 64.21; H, 4.98

IR spectrum (maximum absorptions, $cm^{-1}$, liquid film):

3550-3150, 3060, 3040, 2950-2750, 1760, 1735, 1680, 1660.

$^1$H-NMR ($d_6$-DMSO, $\delta$ ):

4.30(d, 1H), 4.95(s, 2H), 5.20(d, 1H), 5.25(q, 2H), 7.20-7.45(m, 5H). The signals of the OH and COOH protons were too broad to determine.

Reference Example 19

A mixture of 196 g of L-gulosaccharoascorbic acid monohydrate, 5 ml of concentrated hydrochloric acid and 800 ml of methanol was refluxed under heating for 4 hours. After completion of the reaction, low boiling temperature materials were distilled off under reduced pressures, to provide crude methyl L-gulosaccharoascorbate as a viscous liquid. The crude product was dissolved in 800 ml of dimethyl sulfoxide, and there were added to the resultant solution 276 g of potassium carbonate and 242 g of benzyl chloride, followed by stirring at room temperatures for 16 hours.

After completion of the reaction, about 500 ml of water were added to the reaction mixture, and the mixture was extracted three times with dichloromethane in amounts of 3 liters. The extract was dried over anhydrous sodium sulfate, and concentrated by removing the solvent by distillation under reduced pressures. The resultant residue was separated and purified by silica gel chromatography using dichloromethane as a solvent to provide 48.7 g of methyl 3-O-benzyl-L-gulosaccharoascorbate in an overall yield of 17.6 % and 142 g of methyl 2,3-di-O-benzyl-L-gulosaccharoascorbate in an overall yield of 39.2 %, both as oily materials.

The analytical data of these esters are shown below.

Methyl 3-O-benzyl-L-gulosaccharoascorbate:

Elemental analysis (%) for $C_{14}H_{14}O_7$
Calculated:
C, 57.14; H, 4.80
Found:
C, 56.87; H, 4.53
IR spectrum (maximum absorptions, $cm^{-1}$, liquid film):
3600-3100, 3050, 1760, 1690.
$^1$H-NMR ($CDCl_3$, $\delta$ ):
2.94(d, 1H), 3.87(s, 3H), 4.50(m, 1H), 4.88(br, 1H), 4.99(d, 1H), 5.38-5.55(q, 2H), 7.2-7.45(m, 5H).

Methyl 2,3-di-O-benzyl-L-gulosaccharoascorbate:

Elemental analysis (%) for $C_{21}H_{20}O_7$
Calculated:
C, 65.62; H, 5.24
Found:
C, 65.68; H, 5.32
IR spectrum (maximum absorptions, $cm^{-1}$, liquid film):
3600-3200, 3100-2850, 1760, 1680.
$^1$H-NMR ($CDCl_3$, $\delta$ ):
2.95(d, 1H), 3.84(s, 3H), 4.35-4.50(q, 1H), 4.94(d, 1H), 5.09(s, 2H), 5.05-5.35(q, 2H), 7.20-7.40(m, 10H).

Reference Example 20

To a solution of 122 g of methyl 2,3-di-O-benzyl-L-gulosaccharoascorbate in 500 ml of acetonitrile were added 200 ml of 3N hydrochloric acid, and the solution was refluxed under heating for 6 hours.

After completion of the reaction, the reaction mixture was concentrated under reduced pressures, and the residue was purified by silica gel chromatography using chloroform as a solvent to provide 79 g of 2,3-di-O-benzyl-L-gulosaccharoascorbic acid in a yield of 67.2 %.

Reference Example 21

To a mixture of 25.0 g of 2,3-di-O-benzyl-D-glucosaccharoascorbic acid, 20 ml of anhydrous acetic acid and 200 ml of dichloromethane was added one drop of concentrated sulfuric acid, and the mixture was stirred at room temperatures for 15 hours.

After completion of the reaction, low boiling point materials were removed by distillation under reduced pressures, and the resultant residue was subjected to silica gel chromatography using dichloromethane/methanol (95/5) as a solvent to provide 23.75 g of 2,3-di-O-benzyl-5-acetyl-D-glucosaccharoascorbic acid in a yield of 85.3 %.

Melting point: 85-92° C

Elemental analysis (%) for $C_{22}H_{20}O_8$

Calculated:

C, 64.07; H, 4.89

Found:

C, 63.97; H, 4.95

IR spectrum (maximum absorptions, $cm^{-1}$, KBr):

3200, 1770, 1760, 1745, 1660.

$^1$H-NMR ($CDCl_3$, δ ):

2.09(s, 3H), 5.09(s, 2H), 5.12(d, 1H), 5.18(s, 2H), 5.56(d, 1H), 7.05-7.4(m, 10H). The COOH was too broad to determine.

Reference Example 22

To a solution of 4.26 g of methyl 2,3-di-O-benzyl-D-glucosaccharoascorbate in 40 ml of dichloromethane were added 1.81 g of anhydrous acetic acid and then one drop of concentrated sulfuric acid, and the mixture was stirred at room temperatures for 4 hours.

After completion of the reaction, low boiling point materials were removed by distillation under reduced pressures, and the resultant residue was subjected to silica gel chromatography using ethyl acetate/n-hexane (1/2) as a solvent to provide 4.39 g of methyl 2,3-di-O-benzyl-5-O-acetyl-D-glucosaccharoascorbate as an oily material in a yield of 92.9 %. IR spectrum (maximum absorptions, $cm^{-1}$, liquid film):

1780-1740, 1680.

$^1$H-NMR ($CDCl_3$, δ ):

2.11(s, 3H), 3.52(s, 3H), 5.12(s, 1H), 5.14(1H), 5.17(s, 2H), 5.57(d, 1H, J=3Hz), 7.06-7.40(m, 10H).

Mass spectrum (m/e):

426($M^+$), 335, 320, 260.

Reference Example 23

To a solution of 2.06 g of 2,3-di-O-benzyl-5-acetyl-D-glucosaccharoascorbic acid in 30 ml of ether were added in small portions an ether solution of diazomethane. The reaction was stopped until the yellow color of diazomethane came to remain in the reaction mixture.

A small amount of acetic acid was added to the reaction mixture to disappear the yellow color, and then the solvent was removed by distillation under reduced pressures, to provide 2.14 g of methyl 2,3-di-O-benzyl-5-O-acetyl-D-glucosaccharoascorbate in a yield of 100 %.

Reference Example 24

To a solution of 10.0 g of 2,3-di-O-benzyl-D-glucosaccharoascorbic acid in 20 ml of dimethylformamide were added 5.58 g of potassium carbonate, and then 14.7 g of tert. -butyl bromide, followed by stirring at 40° C for 16 hours.

After completion of the reaction, 500 ml of ether and 80 ml of water were added to obtain an ether extract. The ether extract was washed with water, dried, and the ether was removed by distillation under reduced pressures. The residue was subjected to silica gel chromatography using dichloromethane/ethyl acetate (1/1) as a solvent. The product was recrystallized from dichloromethane/n-hexane to provide 2.66 g of t-butyl 2,3-di-O-benzyl-D-glucosaccharoascorbate in a yield of 23.1 %.

Melting point: 103-105°C
Elemental analysis (%) for $C_{24}H_{26}O_7$:
Calculated:
C, 67.59; H, 6.15
Found:
C, 67.65; H, 6.17
IR spectrum (maximum absorptions, $cm^{-1}$, KBr):
3500, 1770, 1720, 1680.
$^1$H-NMR ($CDCl_3$, $\delta$ ):
1.33(s, 9H), 3.00(m, 1H), 4.45(t, 1H), 4.94(m, 1H), 5.11 (s, 4H), 7.37(s, 10H).

Reference Example 25

To a solution of 792 mg of 2,3-di-O-benzyl-5-acetyl- D-glucosaccharoascorbic acid in 40 ml of dried dichloromethane were added 844 mg of triphenyldibromophosphine, and the mixture was stirred at room temperatures for 5 minutes. Then 3.76 g of phenol were added to the mixture and the mixture was stirred for 10 minutes, and then there were added 158 mg of pyridine, followed by stirring at room temperatures for another 10 minutes and then standing overnight.

After completion of the reaction, dichloromethane and water were added to obtain an organic extract. The extract was dried, and the solvent was removed by distillation under reduced pressures. The residue was subjected to silica gel chromatography using chloroform as a solvent to provide 720 mg of phenyl 2,3-di-O-benzyl-5-acetyl-D-glucosaccharoascorbate as an oily material in a yield of 73.8 %.
IR spectrum (maximum absorptions, $cm^{-1}$, liquid film):
1780, 1765, 1750, 1675.
$^1$H-NMR ($CDCl_3$, $\delta$ ):
2.17(s, 3H), 5.15(s, 2H), 5.23(s, 2H), 5.30(d, 1H), 5.79 (d, 1H), 6.60-7.40(m, 15H).

Reference Example 26

An amount of 300 mg of phenyl 2,3-di-O-benzyl-5-acetyl-D-glucosaccharoascorbate was dissolved in a mixture of 2N hydrochloric acid and acetonitrile (1/9), and the solution was stirred at 80°C for 4 hours.

After completion of the reaction, acetonitrile was removed by distillation under reduced pressures, and ether and water were added to obtain an ether extract. The extract was dried, and the ether was removed by distillation under reduced pressures. The residue was subjected to silica gel chromatography using chloroform as a solvent to provide 50 mg of phenyl 2,3-di-O-benzyl-D-glucosaccharoascorbate as an oily material in a yield of 18.7 %.
IR spectrum (maximum absorptions, $cm^{-1}$, liquid film):
3650-3200, 1770, 1765, 1675.
$^1$H-NMR ($CDCl_3$, $\delta$ ):
2.93(d, 1H), 4.85(dd, 1H), 5.33-5.92(m, 5H), 6.55-7.43 (m, 15H).

Reference Example 27

To a solution of 3.17 g of 2,3-di-O-benzyl-5-O-acetyl-D-glucosaccharoascorbic acid in 80 ml of dried dichloromethane were added 3.38 g of dibromotriphenylphosphorane, and the mixture was stirred at room temperatures for 10 minutes. Then 9.93 g of p-methoxyphenol was added to the mixture, and the mixture was stiired for 10 minutes, and then there were added gradually 0.65 ml of pyridine, followed by stirring at room temperatures for 1 hour and standing overnight.

After completion of the reaction, the reaction mixture was added to 100 ml of water, and the mixture was extracted three times with dichloromethane. The extract was dried over sodium sulfate, and the solvent was removed by distillation therefrom. The thus obtained residue was subjected to silica gel chromatography using chloroform as a solvent to provide 1.31 g of p-methoxyphenyl 2,3-di-O-benzyl-5-O-acetyl-D-glucosaccharoascorbate as an oily material in a yield of 31.6 %.

IR spectrum (maximum absorptions, $cm^{-1}$, liquid film):
1770-1750, 1680.
$^1$H-NMR ($CDCl_3$, $\delta$ ):
2.16(s, 3H), 3.76(s, 3H), 5.15(s, 2H), 5.22(s, 2H), 5.29 (d, 1H), 5.77(d, 1H), 6.67(s, 4H), 7.10-7.46(m, 10H).

Reference Example 28

An amount of 1.02 g of p-methoxyphenyl 2,3-di-O-benzyl-5-acetyl-D-glucosaccharoascorbate was dissolved in a mixture of 2 ml of 2N hydrochloric acid and 18 ml of acetonitrile, and the solution was stirred at 80 °C for 8 hours.

After completion of the reaction, acetonitrile was removed by distillation, and 80 ml of water were added to the reactiion mixture. The mixture was extracted three times with ether, and the ether extract was dried over magnesium sulfate, followed by removing the ether by distillation. The residue was then subjected to silica gel chromatography using chloroform as a solvent to provide 510 mg of p-methoxyphenyl 2,3-di-O-benzyl-D-glucosaccharoascorbate as an oily material in a yield of 49.0 %.
IR spectrum (maximum absorptions, $cm^{-1}$, liquid film):
3700-3150, 1780-1740, 1675.
$^1$H-NMR ($CDCl_3$, $\delta$ ):
2.95(d, 1H), 3.77(s, 3H), 4.83(dd, 1H), 4.93-5.30(m, 5H), 6.65 (d, 4H), 7.10-7.43 (m, 10H).

Reference Example 29

To a solution of 6.0 g of 2,3-di-O-benzyl-D-glucosaccharoascorbic acid in 35 ml of dimethylformamide were added 1.79 g of potassium carbonate at room temperatures under stirring. After ceasing of generation of carbon dioxide gas, 5.40 g of stearyl bromide were added to the mixture, and the mixture was stirred at 50 °C for 7 hours.

After completion of the reaction, water was added to the reaction mixture, and the mixture was extracted three times with ether in amounts of 500 ml. The ether extract was washed with water, dried over sodium sulfate, and the ether was removed by distillation under reduced pressures. The residue was subjected to silica gel chromatography using ethyl acetate/n-hexane (1/3) as a solvent to provide 8.16 g of n-octadecyl 2,3-di-O-benzyl-D-glucosaccharoascorbate in a yield of 80.9 %.
Melting point: 42-42.5 °C (recrystallized from methanol)
Elemental analysis (%) for $C_{38}H_{54}O_7$:
Calculated:
C, 73.28; H, 8.74
Found:
C, 73.29; H, 8.79
IR spectrum (maximum absorptions, $cm^{-1}$, KBr):
3500, 1770, 1740, 1680.
$^1$H-NMR ($CDCl_3$, $\delta$ ):
0.75-0.95(t, 3H), 1.0-1.6(m, 32H), 2.94(d, 1H), 3.95(t, 2H), 4.5-4.62(m, 1H), 4.95-5.15(m, 1H+4H), 7.0-7.4-(m, 10H).

Reference Example 30

An amount of 5.37 g of dicalcium 2-keto-D-glucarate was suspended in 400 ml of methanol. To the suspension were added 2.4 g of sulfuric acid, and the mixture was refluxed under heating for 90 minutes.

After completion of the reaction, sodium hydrogen carbonate was added to the reaction mixture until the mixture became neutral, and then insoluble calcium sulfate and sodium bicarbonate were removed by filtration. The filtrate was concentrated under reduced pressures, and acetone was added to the concentrate to remove insoluble salts therefrom by filtration. The solvent was removed by distillation under reduced pressures from the filtrate, and water was added to the filtrate, followed by free-drying, to provide 2.1 g of dimethyl 2-keto-D-glucarate as a syrupy material in a yield of 44.5 %.
IR spectrum (maximum absorptions, $cm^{-1}$, liquid film):
3600-3200, 1760-1720, 1630.

$^1$H-NMR ($d_6$-DMSO, δ ):

3.62(s, 3H), 3.65(s, 3H), 3.9-4.2(m, 3H), 5.6(br, 2H), 6.8(br, 1H).

$^{13}$C-NMR ($d_6$-DMSO, δ ):

51.53(q), 52.02(q), 76.64(d), 77.89(d), 78.24(d), 70.03(d), 79.69(d), 82.61(d), 99.99(s), 103.85(s), 168.59-(s), 169.25(s), 169.90(s), 170.35(s).

Example 1

An amount of 100 g of D-glucosaccharoascorbic acid monohydrate was dissolved in 1 liter of methanol. To the solution were added 3 drops of concentrated sulfuric acid, and the mixture was refluxed under heating for 5 hours on a water bath.

After completion of the reaction, low boiling point materials were removed by distillation under reduced pressures, and 300 ml of dichlorometane were added to the concentrate. Crystallization took place gradually. After standing overnight in a refrigerator, the resultant precipitates were collected by filtration, washed with a small amount of ether, and dried under reduced pressures, to provide 101.9 g of methyl D-glucosaccharoascorbate monohydrate in a yield of 95.5 %.

Melting point: 76.5-77.5 °C (recrystallized ftom methanol dichloromethane (1/1)).

Elemental analysis (%) for $C_7H_8O_7 \bullet H_2O$:

Calculated:

C, 37.85; H, 4.54

Found:

C, 37.75; H, 4.57

IR spectrum (maximum absorptions, $cm^{-1}$, KBr):

3560, 3500-3100(br), 1760-1740, 1690.

$^1$H-NMR ($d_6$-DMSO, δ ):

3.63(s, 3H), 4.50(d, 1H, J=3Hz), 4.93(d, 1H, J=3Hz), ca. 6-9(br, 3H).

Example 2

To a mixture of 4.0 g of methyl iodide, 1.46 of D-glucosaccharoascorbic acid monohydrate and 10 ml of dimethyl formamide were added 1.07 g of potassium carbonate under stirring, and the reaction was carried out at room temperatures overnight.

After completion of the reaction, the reaction mixture was concentrated under reduced pressures. An amount of 5 ml of water was added to the concentrate and then the mixture was neutralized with diluted hydrochloric acid. The mixture was then extracted three times with ether in amounts of 300 ml. The ether extract was washed with water, dried over sodium sulfate, and the ether was removed by distillation under reduced pressures to prvide solid materials. The solids were recrystallized from methanol/dichloromethane, to provide 0.47 g of methyl D-glucosaccharoascorbate monohydrate in a yield of 30.2 %.

Example 3

An amount of 4.03 g of methyl 2,3-di-O-benzyl-5-acetyl-D-glucosaccharoascorbate was dissolved in 40 ml of methanol, and there were added thereto 200 mg of 2 % Pd/C, thus carrying out hydrogenation reaction at normal temperatures under normal pressures.

After completion of the reaction, the catalyst was removed from the reaction mixture by filtration, and methanol was removed by distillation, to provide crystalline solids. The solids were recrystallized from ethyl acetate/n-hexane (2/1) to provide 2.18 g of methyl 5-O-acetyl-D-glucosaccharoascorbate in a yield of 93.7 %.

Melting point: 135.5-143 °C

Elemental analysis (%) for $C_9H_{10}O_8$:

Calculated:

C, 43.91; H, 4.09

Found:

C, 43.81; H, 4.04

IR spectrum (maximum absorptions, $cm^{-1}$, KBr):
3370, 3200-2950, 1765, 1730, 1670.
$^1H$-NMR ($d_6$-DMSO, $\delta$ ):
2.11(s, 3H), 3.65(s, 3H), 5.16(d, 1H, J=3Hz), 5.47(d, 1H, J=3Hz). The OH was too broad to determine.

Example 4

An amount of 10.2 g of methyl D-glucosaccharoascorbate monohydrate was dissolved in 150 ml of methanol, and cooled with ice water. There were added dropwise to the solution 1.8 g of a solution of sodium hydroxide in 70 ml of methanol.

After completion of the reaction, methanol was removed from the reaction mixture by distillation under reduced pressures, and 200 ml of acetone were added to the concentrate, to provide precipitates. The precipitates were collected by filtration and dried under reduced pressures, to provide 10.3 g of Na salt of methyl D-glucosaccharoascorbate in a yield of 91.5 %.
Melting point: ca. 110 °C (decomposed)
Elemental analysis (%) for $C_7H_7O_7Na \bullet H_2O$:
Calculated:
C, 34.44; H, 3.72
Found:
C, 34.20; H, 3.80
IR spectrum (maximum absorptions, $cm^{-1}$, KBr):
3700-2800, 1740, 1630-1590.

Example 5

An amount of 10.0 g of methyl D-glucosaccharoascorbate monohydrate was dissolved in 200 ml of methanol. A solution of 6.4 g of barium hydroxide octahydrate in 200 ml of methanol was added dropwise to the solution under stirring. After removing insoluble materials by filtration, the filtrate was concentrated to form precipitates. An amount of 300 ml of acetone was added to the concentrate, the precipitates were collected by filtration, washed with acetonece, and dried, to provide 9.7 g of Ba salt of methyl D-glucosaccharoascorbate in a yield of 74.4 %.
Melting point: ca. 150 °C (decomposed)
Elemental analysis (%) for $C_7H_7O_7Ba \bullet 0.5H_2O$:
Calculated:
C, 29.01; H, 3.13
Found:
C, 28.92; H, 3.09
IR spectrum (maximum absorptions, $cm^{-1}$, KBr):
3700-2500(br), 1750(sh), 1740(sh), 1730, 1720(sh), 1600(very strong).

Example 6

To a solution of 10.0 g of L-gulosaccharoascorbic acid in 400 ml of methanol were added 0.5 ml of concentrated hydrochloric acid, and the mixture was refluxed under heating for 3 hours.

After completion of the reaction, methanol was removed from the reaction mixture by distillation under reduced pressures, to provide pasty materials, which became semi-solid after standing overnight at room temperatures. The materials were dissolved in hot ethyl acetate, and recrystallized therefrom to provide methyl L-gulosaccharoascorbate 0.5 hydrate in a yield of 61.5 %.
Melting point: 100-101 °C (decomposed)
Elemental analysis (%) for $C_7H_8O_7 \bullet 0.5H_2O$:
Calculated:
C, 39.45; H, 4.26
Found:
C, 39.30; H, 4.25

IR spectrum (maximum absorptions, $cm^{-1}$, KBr):
3520, 3500-3000(br), 1765, 1750, 1735, 1690, 1670.
$^1$H-NMR ($d_6$DMS, δ):
3.70(s, 3H), 4.43(br,1H), 4.95(d, 1H, J=3Hz), 5.7(br, 1H). 8.4(br, 1H), 11.1(br, 1H).

Example 7

A mixture of 10.4 g of D-glucosaccharoascorbic acid monohydrate, 30 ml of ethylene glycol and and one drop of sulfuric acid was heated under stirring for 4 hours on an oil bath at 100° C.

One tenth of the resultant reaction mixture was subjected to activated carbon chromatography using acetone/water (1/1) as an eluent, the fractions containing the products were collected, and purified by use of 400 ml of Sefadex G-10. The fractions were then concentrated under reduced pressures, to provide 0.85 g of β-hydroxyethyl D-glucosaccharoascorbate 0.5 hydrate as powders in a yield of 69.9 %.

Elemental analysis (%) for $C_8H_{10}O_8 \bullet 0.5H_2O$:
Calculated:
C, 39.51; H, 4.56
Found:
C, 39.91; H, 4.76
IR spectrum (maximum absorptions, $cm^{-1}$, KBr):
3600-2800, 1770-1740, 1700-1670.
$^1$H-NMR ($d_6$-DMSO, δ):
3.3-3.7(t, 2H), 3.8-4.3(t, 2H), 4.50(d, 1H, J=3Hz), 4.97(d, 1H, J=3Hz), ca. 4-7(br, 3H), ca. 11.0(br, 1H).

Example 8

A mixture of 10.4 g of D-glucosaccharoascorbic acid monohydrate, 200 ml of isopropyl alcohol and one drop of concentrated sulfuric acid was refluxed under stirring and heating for 4 hours.

The resultant reaction mixture was concentrated under reduced pressures, and the residue was dissolved in 500 ml of ethyl acetate and washed with water. After drying over sodium sulfate, the solvent was removed from the mixture by distillation under reduced pressures, and the residue was subjected to silica gel chromatography using ethyl acetate/dichloromethane as a solvent to provide 8.5 g of isopropyl D-glucosaccharoascorbate monohydrate as an oily material in a yield of 67.8 %.

Elemental analysis (%) for $C_9H_{12}O_7 \bullet H_2O$:
Calculated:
C, 43.20; H, 5.64
Found:
C, 43.21; H, 5.59
IR spectrum (maximum absorptions, $cm^{-1}$, liquid film):
3600-3000(br), 1760(sh), 1740, 1720, 1690.
$^1$H-NMR ($d_6$-DMSO, δ):
1.05-1.3.(,6H), 4.03(q, 1H, J=7Hz), 4.43(d, 1H, J=3Hz), 4.87(d, 1H, J=3Hz), 6.4-8.5(br, 3H), ca. 11.0(br, 1H).

Example 9

To a mixture of 3.4 g of isopropyl iodide, 2.08 g of D-glucosaccharoascorbic acid monohydrate and 30 ml of dimethyl sulfoxide were added under stirring 1.52 g of potassium carbonate, and the reaction was carried out at room temperatures for 15 hours.

After completion of the reaction, the reaction mixture was added to 70 ml of water, neutralized with diluted hydrochloric acid, and extracted three times with ethyl acetate in total of 500 ml. The extract was washed with water, dried over anhydrous sodium sulfate, and the solvent was removed by distillation under reduced pressures. The residue was then subjected to silica gel chromatography using ethyl acetate as a solvent to provide 0.25 g of isopropyl D-glucosaccharoascorbate monohydrate in a yield of 10.0 %.

Example 10

A mixture of 10.4 g of D-glucosaccharoascorbic acid, 200 ml of allyl alcohol and one drop of concentrated sulfuric acid was refluxed under heating on an oil bath at 105-110 °C for 6 hours.

After completion of the reaction, the reaction mixture was concentrated under reduced pressures, and the residue was subjected to silica gel chromatography using ethyl acetate as a solvent to provide 10.1 g of allyl D-glucosaccharoascorbate as an oily material in a yield of 87.7 %.

Elemental analysis (%) for $C_9H_{10}O_7$:
Calculated:
C, 46.96; H, 4.38
Found:
C, 47.24; H, 4.70
IR spectrum (maximum absorptions, $cm^{-1}$, liquid film):
3600-2800(br), 1760-1680(br).
$^1$H-NMR ($d_6$-DMSO, $\delta$):
4.4-4.7(m, 3H), 4.92(d, 1H, J=3Hz), 5.1-5.5(m, 2H), 5.5-6.2(m, 1H +1H), ca. 8.3(br, 1H), ca. 11.1(br, 1H).

Example 11

To a mixture of 10.4 g of D-glucosaccharoascorbic acid monohydrate, 25 ml of propargyl alcohol and one drop of concentrated sulfuric acid was refluxed under heating on an oil bath at 120-130 °C for 4 hours.

After completion of the reaction, low boiling temperature materials were removed by distillation under reduced pressures. The residue was subjected to silica gel chromatography using ethyl acetate as a solvent to provide 8.3 g of propargyl D-glucosaccharoascorbate 0.5 hydrate as an oily material in a yield of 70.0 %.

Elemental analysis (%) for $C_9H_8O_7 \bullet 0.5H_2O$:
Calculated:
C, 45.58; H, 3.82
Found:
C, 45.74; H, 4.13
IR spectrum (maximum absorptions, $cm^{-1}$, liquid film):
3600-2800(br), 1770-1740(br), 1730-1670(br).
$^1$H-NMR ($d_6$-DMSO, $\delta$):
3.5-3.35(,1H), 4.57(d, 1H, J=3Hz), 4.6-4.8(2H), 4.95(d, 1H, J=3Hz), 5.0-6.6(br, 1H), ca. 8.(br, 1H), ca. 11.1(br, 1H).

Example 12

A mixture of 10.4 g of D-glucosaccharoascorbic acid monohydrate, 50 ml of isobutanol and one drop of concentrated sulfuric acid was refluxed with stirring under heating for 4 hours.

After completion of the reaction, the reaction mixture was concentrated under reduced pressures, and the residue was dissolved in 500 ml of ethyl acetate, washed with water, and dried over anhydrous sodium sulfate. After removing the solvent by distillation, the residue was recrystallized from ether/ethyl acetate, to provide 9.0 g of isobutyl D-glucosaccharoascorbate in a yield of 73.1 %.

Melting point: 131-132 °C
Elemental analysis (%) for $C_{10}H_{14}O_7$:
Calculated:
C, 48.78; H, 5.73
Found:
C, 48.77; H, 5.79
IR spectrum (maximum absorptions, $cm^{-1}$, KBr):
3600-3000, 1760, 1750(sh), 1710, 1685.
$^1$H-NMR ($d_6$-DMSO, $\delta$):
0.90(d, 6H, J=6Hz), 1.65-2.05(m, 1H), 3.86(d, 2H, J=6Hz), 4.51(d, 1H, J=3Hz), 4.95(d, 1H, J=3Hz). The OH was too broad to determine.

Example 13

An amount of 2.38 g of tert.-butyl 2,3-di-O-benzyl-D-glucosaccharoascorbate was dissolved in 80 ml of ethyl actate, and there were added to the solution 200 mg of 5 % Pd/C, and then hydrogenation reaction was carried out at normal temperatures under normal pressures.

After completion of the reaction, the catalyst was removed from the reaction mixture by filtration. The ethyl acetate was removed by distillation from the filtrate, and the residue was recrystallized from dichloromethane/n-hexane, to provide 1.0 g of tert.-butyl D-glucosaccharoascorbate 0.2 hydrate in a yield of 72.8 %.

Melting point: 53-55 °C
Elemental analysis (%) for $C_{10}H_{14}O_7 \bullet 0.2H_2O$:
Calculated:
C, 48.43; H, 5.81
Found:
C, 48.24; H, 5.86
IR spectrum (maximum absorptions, $cm^{-1}$, KBr):
1770, 1730, 1680.
$^1$H-NMR ($d_6$-DMSO, $\delta$):
1.37(s, 9H), 4.35(br, 1H), 4.86(d, 1H), 5.60(br, 1H), 8.37(br, 1H), 11.02(br, 1H).

Example 14

A mixture of 10.4 g of D-glucosaccharoascorbic acid, 50 g of cyclohexanol and one drop of concentrated sulfuric acid was heated at 100 °C with stirring for 3 hours.

After completion of the reaction, low boiling temperature materials were removed by distillation under reduced pressures of not more than 3 mmHg. The residue was dissolved in 400 ml of ethyl acetate, washed with water, and dried over anhydrous sodium sulfate. After removing the solvent by distillation under reduced pressures, the residue was subjected to silica gel chromatography using dichloromethane/isopropyl ether as a solvent to provide 11.25 g of cyclohexyl D-glucosaccharoascorbate in a yield of 82.6 %.

Melting point: 112-114 °C (recrystallized from ethyl acetate/n-hexane)
Elemental analysis (%) for $C_{12}H_{16}O_7$:
Calculated:
C, 52.94; H, 5.92
Found:
C, 53.31; H, 5.95
IR spectrum (maximum absorptions, $cm^{-1}$, KBr):
3600-3000, 1765, 1740, 1700.
$^1$H-NMR ($d_6$-DMSO, $\delta$):
1.0-1.9(br, 10H), 4.44(d, 1H), 4.5-4.9(br, 1H), 4.90(d, 1H), 6.54(br, 1H), 8.4(br, 1H), 11.5(br,1H).

Example 15

An amount of 50 mg of phenyl 2,3-di-O-benzyl-D-glucosaccharoascorbate was dissolved in 20 ml of ethyl actate, and there were added to the solution 5 mg of 5 % Pd/C, to carry out hydrogenation under normal temperatures and pressures.

After completion of the reaction, the catalyst was removed from the reaction mixture by filtration. The ethyl acetate was removed by distillation from the filtrate, and the residue was subjected to silica gel chromatography using ethyl acetate as a solvent to provide 24 mg of phenyl D-glucosaccharoascorbate as an oily material in a yield of 82 %.

Elemental analysis (%) for $C_{12}H_{10}O_7$:
Calculated:
C, 56.70; H, 3.96
Found:
C, 57.02; H, 4.14

IR spectrum (maximum absorptions, $cm^{-1}$, liquid film):
3680, 3000, 1780, 1760, 1680.
$^1H$-NMR ($d_6$-DMSO, $\delta$):
4.82(d, 1H), 5.13(d, 1H), 6.20(br, 1H), 6.60-7.58(m, 5H), 8.50(br, 1H), 11.53(br, 1H).

Example 16

A mixture of 20.8 g of D-glucosaccharoascorbic acid monohydrate, 54.1 g of benzyl alcohol and one drop of concentrated sulfuric acid was heated under reduced pressures of 20-30 mmHg on an oil bath at about 80 °C for 4 hours.

After completion of the reaction, excess amounts of benzyl alcohol were removed by distillation under reduced pressures of 1-2 mmHg. The residue was then subjected to silica gel chromatography using chloroform/ethyl acetate as a solvent to provide 19.9 g of benzyl D-glucosaccharoascorbate in a yield of 67.8 %.
Melting point: 145-146 °C (recrystallized from acetonitrile)
Elemental analysis (%) for $C_{13}H_{12}O_7$:
Calculated:
C, 55.72; H, 4.32
Found:
C, 55.61; H, 4.32
IR spectrum (maximum absorptions, $cm^{-1}$, KBr):
3600-2800(br), 1770, 1745, 1680.
$^1H$-NMR ($d_6$-DMSO, $\delta$):
4.58(d, 1H, J=3Hz), 4.94(d, 1H, J=3Hz), 5.08(2H), 7.2-7.45(5H), 7.4-7.8(br, 1H), 8.4(br, 1H), 11.2(br, 1H).

Example 17

An amount of 510 mg of p-methoxyphenyl 2,3-di-O-benzyl-D-glucosaccharoascorbate was dissolved in 30 ml of ethyl actate. There were added to the solution 50 mg of 5 % Pd/C, and a hydrogenation reaction was carried out at normal temperatures and under normal pressures.

After completion of the reaction, the catalyst was removed from the reaction mixture by filtration. The ethyl acetate was removed by distillation from the filtrate, to provide 260 mg of p-methoxyphenyl D-glucosaccharoascorbate as a pasty material in a yield of 73.0 %.
IR spectrum (maximum absorptions, $cm^{-1}$, liquid film):
3650-3000, 1780-1750, 1690.
$^1H$-NMR ($d_6$-DMSO, $\delta$):
3.75(s, 3H), 4.77(d, 1H), 5.10(d, 1H), 6.95(s, 4H), 8.45(br, 1H), 11.35(br, 1H). The OH was too broad to determine.

Example 18

A mixture of 5.0 g of methyl D-glucosaccharoascorbate monohydrate, 50 ml of n-octyl alcohol and one drop of concentrated sulfuric acid was heated under stirring on an oil bath at 110-115 °C for 4 hours.

After completion of the reaction, excess amounts of n-octyl alcohol were removed by distillation under reduced pressures of not more than 3 mmHg. The residue was dissolved in 500 ml of ethyl acetate, washed with water, and dried over anhydrous sodium sulfate. After removing the solvent by distillation under reduced pressures, the residue was subjected to silica gel chromatography using ethyl acetate/tetrahydrofuran as a solvent to provide 4.9 g of n-octyl D-glucosaccharoascorbate in a yield of 72.1 %.
Melting point: 89-90 °C (recrystallized from ethyl acetate/n-hexane)
Elemental analysis (%) for $C_{14}H_{22}O_7$:
Calculated:
C, 55.62; H, 7.33
Found:
C, 55.59; H, 7.33

IR spectrum (maximum absorptions, $cm^{-1}$, KBr):
3600-3000(br), 1780, 1760(sh), 1730, 1705, 1690.
$^1$H-NMR ($CDCl_3$, $\delta$):
0.75-1.0(3H), 2.1-2.7(12H), 4.23(t, 2H), 4.79(d, 1H), J=3Hz), 5.13(d, 1H, J=3Hz). The OH was too broad to determine.

Example 19

An amount of 10.0 g of D-glucosaccharoascorbic acid monohydrate was added to 200 ml of butoxyethoxyethyl alcohol, and there was added to the mixture one drop of concentrated sulfuric acid. The resultant mixture was stirred under reduced pressures for 6 hours while water was removed by distillation at 100 °C.

After completion of the reaction, excess amounts of butoxyethoxyethyl alcohol were removed by distillation under reduced pressures. The residue was subjected to silica gel chromatography using dichloromethane/ethyl acetate (1/1) as a solvent to provide 8.77 g of butoxyethoxyethyl D-glucosaccharoascorbate as an oily material in a yield of 54.7 %.

Elemental analysis (%) for $C_{14}H_{22}O_9 \bullet 0.3H_2O$:
Calculated:
C, 49.50; H, 6.70
Found:
C, 49.67; H, 7.01
IR spectrum (maximum absorptions, $cm^{-1}$, liquid film):
3600-3000, 2950, 1750, 1690.
$^1$H-NMR ($d_6$-DMSO, $\delta$):
1.00-0.73(m, 3H), 1.10-1.70(m, 4H), 3.10-3.70(m, 8H), 4.03-4.26(m, 2H), 4.51(d, 1H), 4.93(d, 1H), 5.88(br, 1H), 8.37 (br, 1H), 11.05(br, 1H). The OH was too broad to determine.

Example 20

To a mixture of 5.0 g of phenacyl bromide, 5.2 g of D-glucosaccharoascorbic acid monohydrate and 100 ml of dimethyl formamide were added under stirring 3.8 g of potassium carbonate, and the reaction was carried out at room temperatures for 15 hours.

After completion of the reaction, the reaction mixture was concentrated under reduced pressures. To the residue was added 10 ml of water, and the mixture was neutralized with diluted hydrochloric acid, and extracted three times with ethyl acetate in amounts of 300 ml. The extract was washed with water, dried, and the solvent was removed by distillation under reduced pressures. The residue was recrystallized from ethyl acetate/chloroform (2/3) to provide 0.58 g of phenacyl D-glucosaccharoascorbate 0.4 hydrate in a yield of 7.3 %.

Melting point: 158-160 °C
Elemental analysis (%) for $C_{14}H_{12}O_8 \bullet 0.4H_2O$:
Calculated:
C, 53.31; H, 4.09
Found:
C, 53.35; H, 4.35
IR spectrum (maximum absorptions, $cm^{-1}$, KBr):
3600-2800, 1765, 1750, 1700.
$^1$H-NMR ($d_6$-DMSO, $\delta$):
4.65(d, 1H, J=3Hz), 5.03(d, 1H, J=3Hz), 4.5-5.6(br, 1H), 5.3-5.7(2H), 7.4-8.1(m, 5H+1H), ca. 11.1(br, 1H).

Example 21

A mixture of 41.6 g of D-glucosaccharoascorbic acid monohydrate, 220 ml of n-decyl alcohol and one drop of concentrated sulfuric acid was heated under stirring under reduced pressures of 20-30 mmHg on an oil bath at 110-120 °C for 2 hours.

After completion of the reaction, the reaction mixture was dissolved in 1 liter of ethyl acetate, washed with water, and dried over anhydrous sodium sulfate. After removing low boiling temperature materials by distillation under reduced pressures, excess amounts of n-decyl alcohol were removed by distillation under highly reduced pressures of not more than 2 mmHg. The residue was recrystallized from ethyl acetate/n-hexane to provide 51.4 g of n-decyl D-glucosaccharoascorbate in a yield of 77.8 %.

Melting point: 95-96 °C
Elemental analysis (%) for $C_{16}H_{26}O_7$:
Calculated:
C, 58.17; H, 7.93
Found:
C, 58.37; H, 7.94
IR spectrum (maximum absorptions, $cm^{-1}$, KBr):
3550-3050, 1775, 1740, 1718.
$^1$H-NMR ($d_6$-DMSO, $\delta$):
1.75-1.0(3H), 1.1-1.8(br, 16H), 4.03(t, 2H), 4.48(d, 1H, J=3Hz), 4.92(d, 1H, J=3Hz), 5.0-6.0(br, 1H), ca. 8.3 (br, 1H), ca. 11.15(br, 1H).

Example 22

A mixture of 41.6 g of L-gulosaccharoascorbic acid, 300 ml of n-decyl alcohol and one drop of concentrated sulfuric acid was heated at 110 °C under stirring for 3 hours while the by-produced water was removed by distillation under reduced pressures.

Immediately after completion of the reaction, the reaction mixture was poured into 2 liters of n-hexane, and the resultant precipitates were collected with excess amounts of n-decyl aocohol removed. The precipitates were dissolved in 200 ml of ethyl acetate, and the solution was added to 800 ml of n-hexane. The resultant precipitates were collected, and dissolved in 500 ml of acetonitrile. To the solution was added n-hexane, and the separated acetonitrile layer was dried to provide 49.5 g of n-decyl L-gulosaccharoscorbate in a yield of 74.9 %.

Melting point: 67-70 °C
Elemental analysis (%) for $C_{16}H_{26}O_7$:
Calculated:
C, 58.17; H, 7.93
Found:
C, 58.46; H, 8.21
IR spectrum (maximum absorptions, $cm^{-1}$, KBr):
3550-3100(br), 2930, 2860, 1775, 1750, 1705, 1660.
$^1$H-NMR ($d_6$-DMSO, $\delta$):
0.85(t, 3H), 1.25(s, 16H), 4.12(t, 2H), 4.40(d, 1H), 4.94(d,1H), ca. 5.7(br, 1H), 8.41(br, 1H), 11.0(br, 1H).

Example 23

A mixture of 5.0 g of methyl D-glucosaccharoascorbate monohydrate, 16.8 g of n-dodecyl alcohol and one drop of concentrated sulfuric acid was heated under stirring for 4 hours on an oil bath at 110-115 °C.

After completion of the reaction, the reaction mixture was poured into 100 ml of n-hexane, and the resultant precipitates were collected. After drying, the precipitates were passed through a silica gel column of about 10 cm in length using ethyl acetate/tetrahydrofuran as a solvent. The removal of the solvent from the eluate provided 6.6 g of n-dodecyl D-glucosaccharoascorbate in a yield of 81.4 %.

Melting point: 101-102 °C (recrystallized from ethyl acetate/n-hexane
Elemental analysis (%) for $C_{18}H_{30}O_7$:
Calculated:
C, 60.32; H, 8.44
Found:
C, 60.30; H, 8.47
IR spectrum (maximum absorptions, $cm^{-1}$, KBr):

3600-3050(br), 1770, 1740, 1710.

$^1$H-NMR ($d_6$-DMSO, $\delta$):

0.75-0.95(3H), 2.1-2.7(20H), 4.02(t, 2H), 4.46(d, 1H, J=3Hz), 4.89(d, 1H, J=3Hz), 7.5-9(very br, 2H), ca. 11.1 (br, 1H).

Example 24

A mixture of 10.4 g of D-glucosaccharoascorbic acid monohydrate, 21.4 g of n-tetradecanol and one drop of concentrated sulfuric acid was heated under stirring under reduced pressures of 20-30 mmHg for 3 hours on an oil bath at about 110 °C.

After completion of the reaction, the reaction mixture was poured into 100 ml of n-hexane, and the resultant precipitates were collected. After washing with petroleum ether and drying, the precipitates were recrystallized from ethyl acetate/n-hexane to provide 14.0 g of n-tetradecyl D-glucosaccharoascorbate in a yield of 72.4 %.

Melting point: 110-111 °C

Elemental analysis (%) for $C_{20}H_{34}O_7$:

Calculated:

C, 62.16; H, 8.87

Found:

C, 62.13; H, 8.90

IR spectrum (maximum absorptions, $cm^{-1}$, KBr):

3600-3050(br), 1780(sh), 1770, 1740, 1710.

$^1$H-NMR ($d_6$-DMSO, $\delta$):

0.75-0.95(3H), 2.15-2.65(24H), 4.02(t, 2H), 4.46(br, 1H, J=3Hz), 4.92(d, 1H, J=3Hz), 11.1(br, 1H). The OH was too broad to determine.

Example 25

An amount of 40 g of n-hexadecyl alcohol was placed in a four necked flask and heated on an oil bath at about 80 °C to melt the alcohol. An amount of 10.0 g of methyl D-glucosaccharoascorbate monohydrate and two drops of concentrated sulfuric acid were added to the melt with stirring, and the mixture was stirred for 5 hours under stirring on an oil bath at about 110 °C.

After completion of the reaction, the reaction mixture was poured into 200 ml of n-hexane, and the resultant precipitates were collected. After washing with petroleum ether and drying, the precipitates were recrystallized from ethyl acetate/n-hexane to provide 10.1 g of n-hexadecyl D-glucosaccharoascorbate in a yield of 54.1 %.

Melting point: 111-112 °C

Elemental analysis (%) for $C_{22}H_{38}O_7$:

Calculated:

C, 63.74; H, 9.24

Found:

C, 63.72; H, 9.20

IR spectrum (maximum absorptions, $cm^{-1}$, KBr):

3500-3050(br), 1765, 1740, 1710.

$^1$H-NMR ($d_6$-DMSO, $\delta$):

1.75-1.95(3H), 2.0-2.7(28H), 4.03(t, 2H), 4.47(d, 1H, J=3Hz), 4.90(d, 1H, J=3Hz), ca. 11(br, 1H). Two OH were too broad to determine.

Example 26

An amount of 70 g of n-octadecyl alcohol was placed in a four necked flask and heated on an oil bath at about 80 °C to melt the alcohol, and to this melt were added 41.6 g of D-glucosaccharoascorbate acid monohydrate with stirring. After fully mixing, two drops of concentrated sulfuric acid were added to the mixture, and the mixture was stirred under reduced pressures of 20-30 mmHg on an oil bath at about 110 °C. As the reaction proceeded, the water generated was distilled off

After 3 hour reaction, the reaction mixture was cooled to room temperatures to become solids. The solids were recrystallized from ethyl acetate/n-hexane (1/4) to provide 61.0 g of the first crystals. The mother liquor was concentrated under reduced pressures, and the residue was recrystallized from ethyl acetate/n-hexane (1/5) to provide 7.5 g of the second crystals. Thus, 68.5 g of n-octadecyl D-glucosaccharoascorbate were obtained in total. The yield was found 77.4 %.

Melting point: 118° C (recrystallized from ethyl acetate/n-hexane)

Elemental analysis (%) for $C_{24}H_{42}O_7$:

Calculated:

C, 65.13; H, 9.56

Found:

C, 65.22; H, 9.60

IR spectrum (maximum absorptions, $cm^{-1}$, KBr):

3500-3100, 1770, 1740, 1715.

$^1$H-NMR ($d_6$-DMSO, $\delta$):

0.86(t, 3H), 2.1-2.4(br, 30H), 2.4-2.7(br, 2H), 4.04(t, 2H), 4.46(d, 1H, J=3Hz), 4.90(d, 1H, J=3Hz), 8.1-8.5-(br, 2H), 11.06(br, 1H).

Example 27

A mixture 22.2 g of methyl D-glucosaccharoascorbate monohydrate, 54 g of n-octadecyl alcohol and two drops of concentrated sulfuric acid was stirred under reduced pressures of 20-30 mmHg on an oil bath at about 110° C. As the reaction proceeded, the water and methanol generated were distilled off.

After five hour reaction, the reaction mixture was added to 600 ml of n-hexane with stirring to become solids. The solid materials were collected by filtration, and washed with hot n-hexane. After drying, the solids were dissolved in a least amount of ethyl acetate under heating. To this solution was added n-hexane in small portions so that flake like white crystals precipitated. The precipitates were collected by filtration and dried, to provide 29.3 g of n-octadecyl D-glucosaccharoascorbate in a yield of 66.2 %.

Example 28

To a mixture of 19.0 g of stearyl iodide, 10.4 g of D-glucosaccharoascorbic acid monohydrate and 200 ml of dimethyl formamide were added under stirring 7.60 g of potassium carbonate, and the reaction was carried out at room temperatures for 20 hours.

After completion of the reaction, the reaction mixture was concentrated under reduced pressures. To the residue was added 50 ml of water, and the mixture was neutralized with diluted hydrochloric acid, and extracted three times with ether in amounts of 700 ml. The extract was washed with water, dried over anhydrous sodium sulfate, and the solvent was removed by distillation under reduced pressures. The residue was recrystallized from eter/n-hexane to provide 2.43 g of n-octadecyl D-glucosaccharoascorbate in a yield of 11.0 %.

Example 29

An amount of 7.75 g of n-octadecyl 2,3-di-O-benzyl-D-glucosaccharoascorbate was dissolved in 50 ml of ethyl actate, and there were added to the solution 500 mg of 5 % Pd/C. Then the mixture was stirred under normal pressures in the presence of hydrogen gas for 4 hours and 20 minutes.

After completion of the reaction, the catalyst was removed from the reaction mixture by filtration, washed with small amounts of ethyl acetate, and the washings were combined with the filtrate. The ethyl acetate was removed by distillation from the filtrate under reduced pressures, and the residue was recrystallized from ethyl acetate/n-hexane, to provide 5.17 g of n-octadecyl D-glucosaccharoascorbate in a yield of 93.9 %.

Example 30

A solution of 1.65 g of sodium hydroxide in 100 ml of methanol was added dropwise to a solution of 18.7 g of n-octadecyl D-glucosaccharoascorbate in 100 ml of methanol while the mixture was cooled so that the temperatures of the mixture was maintained at not more than 10 °C.

The resultant white crystals were collected by filtration, washed with methanol and dried to provide 16.5 g of monosodium salt of n-octadecyl D-glucosaccharoascorbate in a yield of 88.2 %.
Melting point: 170-173 °C (decomposition)
Elemental analysis (%) for $C_{24}H_{41}O_7Na$:
Calculated:
C, 62.05; H, 8.90
Found:
C, 62.08; H, 9.21
IR spectrum (maximum absorptions, $cm^{-1}$, KBr):
3600-3350(br), 1760, 1735, 1630.

Example 31

A solution of 1.94 g of 85 % potassium hydroxide in 100 ml of methanol was added dropwise to a solution of 13.3 g of n-octadecyl D-glucosaccharoascorbate in 200 ml of methanol at temperatures of not more than 10 °C.

The resultant precipitates were collected by filtration, washed with methanol and dried to provide 12.0 g of monopottasium salt of n-octadecyl D-glucosaccharoascorbate in a yield of 83.0 %.
Melting point: 120-140 °C (decomposed, not clear)
Elemental analysis (%) for $C_{24}H_{41}O_7K$:
Calculated:
C, 59.97; H, 8.60
Found:
C, 60.33; H, 8.82
IR spectrum (maximum absorptions, $cm^{-1}$, KBr):
3500-3100, 1780, 1760, 1740, 1670, 1640, 1630.

Example 32

A solution of 3.9 g of barium hydroxide octahydrate in 100 ml of methanol was added dropwise to a solution of 13.3 g of n-octadecyl D-glucosaccharoascorbate in 200 ml of methanol at temperatures of not more than 10 °C.

The mixture was stirred to become turbid and finally pasty. The insoluble materials were collected by filtration, washed with methanol and dried to provide 12.0 g of monobarium salt of n-octadecyl D-glucosaccharoascorbate.

The yield was found 93.5 % based on the barium hydroxide octahydrate used.
Melting point: 145-180 °C (decomposed, not clear)
Elemental analysis (%) for $C_{24}H_{41}O_7Ba \bullet 0.5H_2O$:
Calculated:
C, 55.51; H, 8.15
Found:
C, 55.28; H, 8.02
IR spectrum (maximum absorptions, $cm^{-1}$, KBr):
3400-3000, 1765, 1740, 1620.

Example 33

Ammonia was introduced into a solution of 20.0 g of n-octadecyl D-glucosaccharoascorbate in 350 ml of methanol at temperatures about 10 °C. After the introduction of nearly the theoretical amount of ammonia, the insoluble materials formed were collected by filtration, washed with methanol, and dried to provide 17.9

g of monoammonium salt of n-octadecyl D-glucosaccharoascorbate in a yield of 86.2 %.
Melting point: 138-142 °C (decomposed)
Elemental analysis (%) for $C_{24}H_{45}O_7$:
Calculated:
C, 62.72; H, 9.87
Found:
C, 62.66; H, 9.93
IR spectrum (maximum absorptions, $cm^{-1}$, KBr):
3500-3000, 2350, 1760, 1730, 1720(sh), 1620.

Example 34

An amount of 140 g of n-octadecyl alcohol and 0.1 ml of concentrated sulfuric acid were added to 83.2 g of L-gulosaccharoascorbic acid, and the mixture was heated at 100-120 °C for 6 hours with stirring while the water generated by the esterification reaction was removed by distillation under reduced pressures.

After compeltion of the reaction, 2 liters of ethyl acetate and 1 liter of water were added to the reaction mixture, and funnel separation was conducted to remove the unreacted raw materials which transferred to an aqueous layer. An amount of 5 liters of n-hexane was added to the ethyl acetate layer, and the resultant precipitates were collected by filtration and dried, to provide 84 g of n-octadecyl L-gulosaccharoascorbate in a yield of 46.5 %.
Melting point: 94-96 °C
Elemental analysis (%) for $C_{24}H_{42}O_7 \bullet 0.5H_2O$:
Calculated:
C, 63.83; H, 9.60
Found:
C, 64.07; H, 9.61
IR spectrum (maximum absorptions, $cm^{-1}$, KBr):
3500, 3400-3200, 2920, 2850, 1770, 1740, 1705, 1680, 1660, 1640.
$^1$H-NMR ($d_6$-DMSO, $\delta$):
0.85(s, 3H), 1.23(s, 32H), 4.11(t, 2H), 4.4(d, 1H, J=3Hz), 4.95(d, 1H, J=3Hz). The OH was too broad to determine.

Example 35

A mixture of 10.0 g of D-glucosaccharoascorbic acid monohydrate, 16 ml of isostearyl alcohol, one drop of concentrated sulfuric acid, and 30 ml of tetrahydrofuran was refluxed under heating for 7 hours.

After completion of the reaction, the reaction mixture was dissolved in 400 ml of ethyll acetate, washed with water, dried and then concentrated under reduced pressurers. The residue was subjected to silica gel chromatography using n-hexane/ether as a solvent to provide 9.69 g of isostearyl D-glucosaccharoascorbate 0.3 hydrate as an oily material in a yield of 45.0 %.
Elemental analysis (%) for $C_{24}H_{42}O_7 \bullet 0.3H_2O$:
Calculated:
C, 64.35; H, 9.58
Found:
C, 64.28; H, 9.35
IR spectrum (maximum absorptions, $cm^{-1}$, liquid film):
3600-3000, 1760, 1740, 1690.
$^1$H-NMR ($d_6$-DMSO, $\delta$):
1.7-2.1(br, 6H), 2.1-2.45(br, 28H), 2.45-2.8(br, 1H, 4.05(d, 2H), 4.55(d, 1H, J=3Hz), 4.97(d, 1H, J=3Hz). The OH was too broad to determine.

Example 36

A mixture of 20.8 g of D-glucosaccharoascorbic acid monohydrate, 48.6 ml of 65 % oleyl alcohol and two drops of concentrated sulfuric acid was heated on an oil bath at about 110 °C for 2hours under reduced pressures of 20-30 mmHg.

After completion of the reaction, the reaction mixture was dissolved in 500 ml of ethyl acetate, washed with water, dried and then concentrated under reduced pressurers. An amount of about 300 ml of n-hexane was added to the residue, and the resultant precipitates were collected by filtration, and dried to provide 29.8 g of oleyl D-glucosaccharoascorbate in a yield of 67.6 %.

Melting point: 84-85 °C (recrystallized from n-hexane)

Elemental analysis (%) for $C_{24}H_{40}O_7$:

Calculated:

C, 65.43; H, 9.15

Found:

C, 65.41; H, 9.23

IR spectrum (maximum absorptions, $cm^{-1}$, KBr):

3600-3000, 1780, 1765, 1740, 1715, 1700(sh), 1695(sh).

$^1$H-NMR ($d_6$-DMSO, $\delta$):

0.8-1.0(t, 3H), 1.1-2.2(br, 28H), 4.23(t, 2H), 4.79 (d, 1H, J=3Hz), 5.12(d, 1H, J=3Hz), 5.15-5.50(br, 2H). The OH was too broad to determine.

Example 37

A mixture of 1.04 g of D-glucosaccharoascorbic acid monohydrate, 4.61 g of lignoceryl alcohol and one drop of concentrated sulfuric acid was heated on an oil bath at about 120 °C for 2hours under reduced pressures of 20-30 mmHg.

After completion of the reaction, the reaction mixture was cooled. The resultant solids were pulverized and washed with about 100ml of water. After drying and washing with a small amount of n-hexane, the solids were recrystallized from ethyl acetate/n-hexane, to provide 1.93 g of lignoceryl D-glucosaccharoascorbate 1/2 hydrate in a yield of 72.0 %.

Melting point: 118 °C

Elemental analysis (%) for $C_{30}H_{54}O_7 \bullet 0.5H_2O$:

Calculated:

C, 67.26; H, 10.35

Found:

C, 67.41; H, 10.32

IR spectrum (maximum absorptions, $cm^{-1}$, KBr):

3500, 3100, 1760, 1735, 1705.

$^1$H-NMR ($CDCl_3$, $\delta$):

0.98(t, 3H), 1.15-1.45(br, 42H), 1.45-1.60(br, 2H), 4.1(t, 3H), 4.55(d, 1H, J=3Hz), 5.0(d, 1H, J=3Hz). The OH was too broad to determine.

The followings are examples of the electroconductive coating composition of the invention. Parts designate parts by weight.

Example 38

An amount of 30 parts of an acrylic resin was mixed and kneaded with 50 parts copper powder and 20 parts of an organic solvent to provide an electroconductive coating composition. An amount of 10 g of the composition was kneaded with 0.05 g of a saccharoascorbic acid ester as shown in Table 1, and then was mixed with about 5 ml of ethyl acetate to properly adjust the viscosity of the composition. The composition was coated in an area of 7 cm x 7 cm and in a thickness of about 50 micron meter on sheets of glass, and dried overnight.

The surface resistance was measured by use of a tester. Thereafter the surface was forcibly deteriorated in an hot air oven at 80 °C for a predetermined period of time, and then taken out of the oven. After standing to room temperatures, the surface resistance was again measured. The results are shown in Table 1, in which the resistances are an average of four point measurements at a 2 cm interval of terminals

of the tester.

Similarly to the above, the composition was coated in an area of 7 cm x 7 cm and in a thickness of about 100 micron meter on an ABS resin sheet, and dried overnight.

The surface resistance was measured before and after the forced deterioration in the same manner as above. The results are shown in Table 2, in which the resistances are an average of five point measurements at a 1.4 cm interval of terminals of the tester.

For comparison, an electroconductive composition was prepared without the incorporation of the saccharoascorbic acid ester otherwise in the same manner as above. The surface resistances of the glass and ABS resin sheets are shown in Table 1 and 2, respectively.

Table 1

| Additives*) | Surface Resistances (Ω) | | | | | Appearances |
|---|---|---|---|---|---|---|
| | Initial | After (hrs.) | | | | |
| | | 150 | 650 | 986 | 1346 | |
| Methyl ester | 11 | 3 | 1 | 2 | 3 | None |
| Octyl ester | 13 | 3 | 5 | 5 | 3 | None |
| Decyl ester | 12 | 2 | 3 | 3 | 4 | None |
| Cetyl ester | 12 | 3 | 5 | 4 | - | None |
| Oleyl ester | 12 | 4 | 2 | 4 | 4 | None |
| Stearyl ester | 10 | 3 | 3 | 4 | 3 | None |
| Benzyl ester | 10 | 3 | 3 | 3 | 3 | None |
| None | 13 | 6 | 50 | 1000 | - | ** |

*) All are the esters of D-glucosaccharoascorbic acid.
**) The film was found to get dark.

Table 2

| Additives | Surface Resistances (Ω) | | | | Appearances |
|---|---|---|---|---|---|
| | Initial | After (hrs.) | | | |
| | | 360 | 910 | 1340 | |
| Stearyl ester*) | 3 | 2 | 2 | 3 | *** |
| Decyl ester*) | 3 | 3 | 3 | 3.5 | *** |
| Benzyl ester*) | 2 | 2 | 3 | 3 | *** |
| Stearyl ester**) | 3 | 2 | 3 | 3 | *** |
| None | 3 | 10 | 900 | >1KΩ | **** |

*) The esters of D-glucosaccharoascorbic acid.
**) The esters of L-glucosaccharoascorbic acid.
***) The film was found to get slighly dark.
****) The film was found to get dark.

The composition of the invention forms a durable and stable electroconductive layer which is not substantially reduced in electroconductivity with time.

Example 39

An amount of 55 parts of an epoxy resin (Epicoat 828) was mixed and kneaded overnight with 100 parts of copper powder and 100 parts of an organic solvent composed of an equal weight of ethyl acetate and toluene by use of a ball mill, to provide a liquid A. An amount of 5.5 parts of triethylenetetramine was dissolved in 20 parts of a solvent composed of an equal weight of ethyl acetate and toluene, to provide a liquid B.

Then the liquid A was mixed with 0.15 parts of stearyl L-gulosaccharoascorbate, and further with 2.5 parts of the liquid B and 0.1 part of resorcinol, to provide an electroconductive coating composition of the invention.

The composition was coated on an ABS resin sheet in an area of 7 cm x 7 cm and in a thickness of about 100 micron meter with a brush, and dried for 3 days.

The surface resistance was measured by use of a tester. Thereafter the surface was forcibly deteriorated in an hot air oven at 80°C for a predetermined period of time, and then taken out of the oven. After standing to room temperatures, the surface resistance was again measured. The resistances were taken as an average of five point measurements at a 1.4 cm interval of terminals of the tester. The surface resistances were initially 3Ω, and 2Ω and 4Ω, after 455 hours and 960 hours, respectively. No change in the appearances of the coating was observed over the period.

For comparison, an electroconductive coating composition was prepared without the incorporation of the stearyl L-gulosaccharoascorbate otherwise in the same manner as above. In the same experiment as above, the surface resistances were 3Ω, 6Ω and 700Ω, initially, after 455 hours and 960 hours, respectively. The coating was found to get dark after the experiment.

The composition of the invention forms a durable and stable electroconductive layer which is not substantially reduced in electroconductivity with time.

Example 40

An electroconductive coating composition was prepared using benzyl D-glucosaccharoascorbate and otherwise in the same manner as in Example 1. The composition was coated on an ABS resin sheet, dried, and then surface resistance was measured at intervals of time.

. The resistances taken as an average of five point measurements at a 1.4 cm interval of terminals were initially 3Ω, and 2Ω either after 470 hours and 984 hours, respectively. The coating was found to remain unchanged in the appearances.

For comparison, an electroconductive coating composition was prepared using anthranilic acid and urushiol, respectively, and otherwise in the same manner as above. In the same experiment as above, the surface resistances of the coating containing anthranilic acid were initially 4Ω, and 5Ω after 470 hours, but 20Ω after 984 hours. The coating was found unchanged in the appearances.

On the other hand, the surface resistances of the coating containing urushiol were initially 3Ω, and 7Ω after 470 hours, but 15Ω after 984 hours. The coating was found to slightly smell bad.

Example 41

An amount of 112 parts of an acrylic resin (Acrydick A-801 by Dainippon Ink Kagaku Kogyo K.K.) was kneaded with 130 parts of silver powder and 1.5 parts of isobutyl D-glucosaccharoascorbate by use of a ball mill overnight. An amount of 24 parts of the resultant mixture was then admixed with 3.3 parts of an urethane resin (Takenate D-160N by Takeda Chemical Industries, Ltd.) to provide an electroconductive coating composition of the invention.

The composition was coated on an unsaturated polyester resin sheet in an area of 7 cm x 7 cm and in a thickness of about 100 micron meter, dried and aged for 3 days, and the surface resistance was measured with a tester. Thereafter the surface was forcibly deteriorated in an hot air oven at 80°C for a predetermined period of time, and then taken out of the oven. After standing to room temperatures, the surface resistance was again measured.

The resistances taken as an average of five point measurements at a 1.4 cm interval of terminals substantially remained the same over 1500 hours, namely, 1Ω, 1Ω, 2Ω and 1Ω, initially, after 470 hours, 984 hours, and 1500 hours, respectively. Further, no change in the appearances of the coating was observed over the period.

For comparison, an electroconductive coating composition was prepared without the incorporation of the isobutyl D- glucosaccharoascorbate and otherwise in the same manner as above. In the same experiment as above, the surface resistances were initially 3Ω, 12Ω after 470 hours, 600Ω after 984 hours, and more than 1 kΩ after 1500 hours. The coating was observed to get dark during the experiment.

The composition of the invention forms a durable and stable electroconductive layer which is not substantially reduced in electroconductivity with time.

## Claims

1. A saccharoascorbic acid ester or a salt thereof.

2. The saccharoascorbic acid ester or a salt thereof as claimed in claim 1, wherein the saccharoascorbic acid is D-glucosaccharoascorbic acid.

3. The saccharoascorbic acid ester or a salt thereof as claimed in claim 1, which is represented by the general formula of

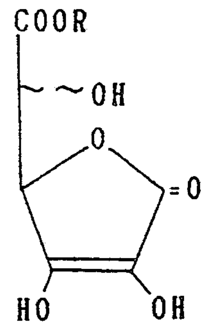

wherein R represents an organic residue of a molecular weight of 15-500, and OH has an S- or an R-configuration.

4. The saccharoascorbic acid ester or a salt thereof as claimed in claim 3, wherein R has a molecular weight of 15-300.

5. The saccharoascorbic acid ester or a salt thereof as claimed in claim 4, wherein R is a hydrocarbon residue of 1-24 carbons.

6. The saccharoascorbic acid ester or a salt thereof as claimed in claim 5, wherein the hydrocarbon residue is an alkyl, an alkenyl, an alkynyl, a cycloalkyl, an aryl or an arylalkyl.

7. The saccharoascorbic acid ester salt as claimed in claim 1, wherein the salt is an alkali metal salt, an alkaline earth metal salt, an ammonium salt, a substituted ammonium salt or a pyridinium salt.

8. A method of producing a saccharoascorbic acid ester represented by the general formula of

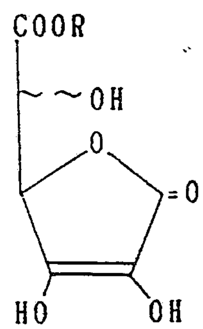

wherein R represents an organic residue of a molecular weight of 15-500, and OH has an S- or an R-configuration, which comprises: reacting a saccharoascorbic acid represented by the formula of

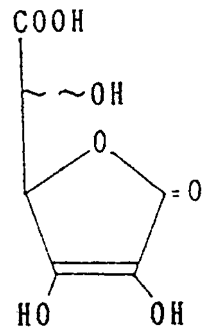

with an alcohol having the general formula of

R-OH

wherein R is the same as before.

9. A method of producing a saccharoascorbic acid ester represented by the general formula of

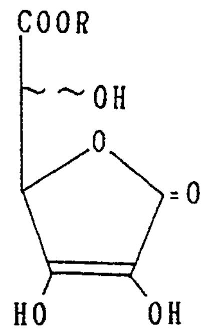

wherein R represents an organic residue of a molecular weight of 15-500, and OH has an S- or an R-configuration, which comprises: reacting a saccharoascorbic acid ester represented by the formula of

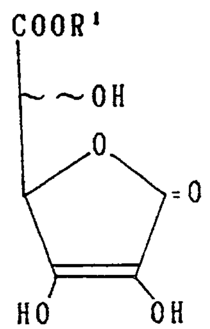

wherein $R^1$ is represents a hydrocarbon residue of 1-12 carbons, with an alcohol having the general formula of

R-OH

wherein R is the same as before.

10. A method of producing a saccharoascorbic acid ester represented by the general formula of

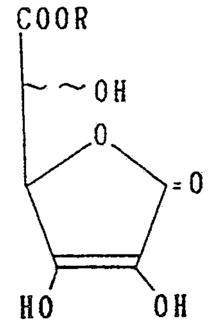

wherein R represents an organic residue of a molecular weight of 15-500, and OH has an S- or an R-configuration, which comprises: reacting a saccharoascorbic acid salt represented by the formula of

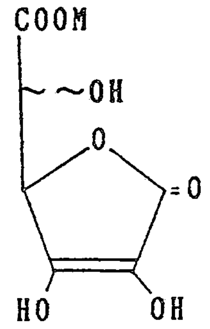

wherein M represents an alkali metal, an alkaline earth metal, an ammonium, a substituted ammonium or pyridinium, with a compound having the general formula of

R-X

wherein R is the same as before and X is a halogen, an alkylsulfonyloxy or an arylsulfonyloxy, or a compound having the general formula of

$(RO)_2SO_2$

wherein R is the same as before.

11. A method of producing a saccharoascorbic acid ester represented by the general formula of

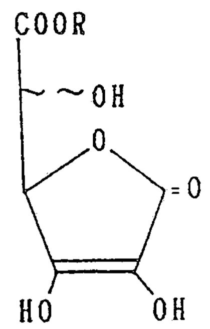

wherein R represents an organic residue of a molecular weight of 15-500, and OH has an S- or an R-configuration, which comprises: subjecting a saccharoascorbic acid ester represented by the general formula of

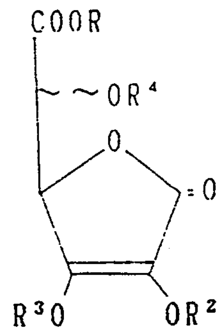

wherein R is the same as before, $R^2$ represents a hydrogen or a hydroxyl protective group, $R^3$ represents a hydroxyl protective group, and $R^4$ represents a hydrogen, a hydroxyl protective group or an acyl group, to an elimination reaction of the hydroxyl protective groups and the acyl group.

12. An electroconductive coating composition which comprises: an organic solvent, a resin and an electroconductive metal powder, and a saccharoascorbic acid ester or a salt thereof.

13. The electroconductive coating composition as claimed in claim 12, wherein R is a hydrocarbon residue of 1-24 carbons.

14. The electroconductive coating composition as claimed in claim 12, which comprises: an organic solvênt, about 20-90 % by weight of a resin based on the composition, about 10-80 % by weight of an electroconducive metal powder, and about 0.01-10 % by weight of a saccharoascorbic acid ester or a salt thereof based on the weight of the metal powder.

15. The electroconductive coating composition as claimed in claim 12, wherein the metal powder is a copper powder.

European Patent Office

# EUROPEAN SEARCH REPORT

Application Number

EP 88 30 5363

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-2 483 251 (N.R. TRENNER)<br>* Whole document *<br>--- | | C 07 D 307/62<br>C 09 D 5/24 |
| A | JOURNAL OF ORGANIC CHEMISTRY, vol. 49, no. 5, 9th March 1984, pages 927-928, American Chemical Society, Easton, US; S. BRANDÄNGE et al.: "Studies on the intramolecular claisen condensation; facile synthesis of tetronic acids"<br>* Whole article, particulary page 928, form. 11 *<br>----- | 1 | |
| | | | TECHNICAL FIELDS SEARCHED (Int. Cl.4) |
| | | | C 07 D 307/00 |

The present search report has been drawn up for all claims

| Place of search | Date of completion of the search | Examiner |
|---|---|---|
| THE HAGUE | 24-08-1988 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document